# DEMANDE DE BREVET EUROPEEN

(11) **EP 1 733 731 A1**
(43) Date de publication de la demande: **20.12.2006**
(21) Numéro de dépôt: 06290946.0
(22) Date de dépôt: 09.06.2006
(51) Int. Cl.: A61K 31/7032, A61K 8/92, A61Q 17/04, A61Q 19/08

(54) **Nouvelles utilisations de derivés du glycerol, notamment dans le domaine cosmétique**

(30) Priorité: 10.06.2005 FR 0505909
(71) Demandeur: AGRO INDUSTRIE RECHERCHES ET DEVELOPPEMENTS (A.R.D.), 51110 Pomacle (FR); Centre National de la Recherche Scientifique, 75794 Paris Cedex 16 (FR); Université De Reims Champagne-Ardenne, 51097 Reims Cedex (FR)
(72) Inventeur: Zeches-Hanrot, Monique, 51430 Bezannes (FR); Hornebeck, William, 02370 Chassemy (FR); Renault, Jean-Hugues, 51100 Reims (FR); Cauchard, Jean-Hubert, 51100 Reims (FR); Delannay, Eldra, 97141 Vieux-Port (FR); Martel, Fréderic, 51100 Reims (FR)
(74) Mandataire: Grosset-Fournier, Chantal Catherine

(57) **Abrégé**

La présente invention concerne l'utilisation d'au moins un composé répondant à la formule (I) suivante : 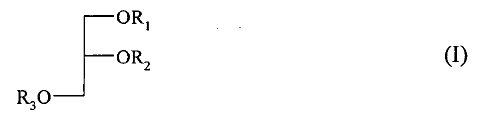 dans laquelle R₁ représente H ou un groupe acyle, R₂ représente notamment H ou un groupe acyle, et R₃ représente H ou un groupe de formule suivante (C) : 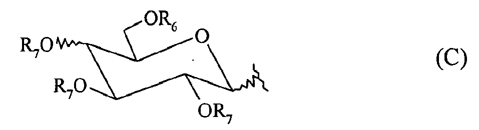 dans laquelle R₆ représente notamment un atome d'hydrogène ou un groupe acyle, et R₇ représente notamment un atome d'hydrogène ou un groupe acyle,
pour la préparation d'une composition cosmétique, d'une composition pharmaceutique destinée au traitement des cancers n'impliquant pas le virus EBV, notamment destinée au traitement des mélanomes, ou destinée au traitement des processus inflammatoires de la sphère buccale, ou d'une composition nutraceutique,

## Description

La présente invention a pour objet de nouvelles utilisations de dérivés du glycérol, tels que des galactosylglycérides, notamment dans le domaine cosmétique.

La présente invention a également pour objet de nouvelles compositions cosmétiques, notamment présentant une activité anti-collagénolytique, et comprenant au moins un glycosylglycéride, de préférence au moins un galactosylglycéride.

La matrice extracellulaire (MEC) est la structure supramoléculaire dans laquelle baignent les cellules des tissus et qui assure la cohésion de ces derniers. Elle est composée d'une substance amorphe formée de polysaccharides (glycosaminoglycanes) et de glycoprotéines (protéoglycanes) et de fibres constituées de protéines structurales (collagènes, élastine, protéines d'adhérence telles que la fibronectine...) dont le type et les proportions varient selon les tissus. Selon sa composition moléculaire, la matrice extracellulaire joue différents rôles physiologiques : cohésion des tissus, soutien mécanique, stockage moléculaire, nutrition... Son renouvellement est essentiel pour la croissance et la réparation des tissus, et intervient aussi dans de nombreux processus pathologiques (cancérogenèse, inflammation, etc...).

Les métalloprotéinases matricielles (MMP) forment une famille de métallo-endopeptidases zinc-dépendantes impliquées dans la dégradation de divers composants de la matrice extracellulaire. Elles jouent un rôle essentiel dans le remodelage tissulaire comme lors de la cicatrisation ainsi que dans des états pathologiques tels que la progression tumorale. Dans le derme, la dégradation des collagènes et de l'élastine est assurée principalement par certaines de ces métalloprotéinases (Mott JD, Werb Z, Curr Opin Cell Biol. 2004 Oct ; 16(5): 558-64. Regulation of matrix biology by matrix metalloproteinases).

Ces métalloprotéinases peuvent être classées en différentes classes selon leurs différences structurales, dont la classe des collagénases interstitielles regroupant notamment la collagénase-1 (MMP-1) et la collagénase-3 (MMP-13), qui ont une activité collagénolytique, la classe des gélatinases regroupant la gélatinase-A (MMP-2) et la gélatinase-B (MMP-9), qui ont une activité de dégradation de l'élastine, et la classe des stromélysines.

L'activité des MMP est notamment contrôlée par des inhibiteurs endogènes connus sous le nom de TIMP ("tissue inhibitors of matrix metalloproteinases"), qui forment un complexe avec les formes actives des MMP et permettent ainsi de maintenir un équilibre physiologique entre la synthèse et la dégradation de la matrice extracellulaire en limitant l'activité des MMP. Ainsi, lors de processus pathologiques, cet équilibre est rompu et il y a surexpression des MMP et non des TIMP : la balance de dégradation se déplace en faveur des MMP et les TIMP sont en défaut. La quantité de TIMP "surexprimable" est alors toujours inférieure à la quantité de MMP surexprimée. Par ailleurs, plusieurs protéases comme la plasmine et les MMP elles-mêmes sont capables d'activer les MMP. Ainsi, l'activation de la plasmine, qui est à l'origine des cascades protéolytiques impliquées dans le vieillissement, entraîne l'activation de la MMP-3 (stromélysine 1) mais également de la MMP-1 (collagénase 1), qui sont des enzymes particulièrement impliquées dans la dégradation de la matrice extracellulaire observée au cours des vieillissements cutanés.

Plus précisément, la collagénase interstitielle ou MMP-1 (EC : 3.4.24.7) clive spécifiquement les trois chaînes α de la triple hélice des collagènes de type I, II et III en une position unique située aux trois-quarts de la molécule par rapport à son extrémité N-terminale (Woessner J.F. et Nagase H., Matrix metalloproteinases and TIMPs. Oxford; New York: Oxford University Press, 2000). La MMP-1 est distribuée de manière ubiquitaire, produite par les fibroblastes, les chondrocytes, les macrophages, les cellules endothéliales, les kératinocytes, et surexprimée par un grand nombre de stimuli.

La gélatinase A ou MMP-2 (EC : 3.4.24.24) tire son nom de sa capacité à dégrader le collagène de type I dénaturé ou gélatine (Murphy G. et Crabbe T., Gelatinases A and B. Methods Enzymol., 1995; 248: 470-84). La MMP-2 est synthétisée sous la forme d'une préproenzyme ; la masse moléculaire apparente de la proenzyme est de 72 kDa et celle de la forme active de 62 kDa. La MMP-2 est distribuée de manière ubiquitaire, exprimée par un grand nombre de cellules en culture. Une augmentation de son expression est observée au niveau du stroma des tumeurs cancéreuses (Polette M., Glibert N., Stas I., Nawrocki B., Noel A., Remacle A., Stetler-Stevenson W.G., Birembaut P. et Foidart M., Gelatinase A expression and localization in human breast cancers. An in situ hybridization study and immunohistochemical detection using confocal microscopy. Virchows Arch 1994; 424(6): 641-5).

La terminologie de stromélysine-1 (MMP-3, EC : 3.4.24.17) désigne la métalloprotéinase des cellules stromales capable de dégrader les protéines de la MEC. La MMP-3 dégrade un grand nombre de protéine de la matrice extracellulaire (MEC) : aggrécane, fibronectine, laminine, collagènes de type III, IV, IX et X, télopeptides du collagène de type I, ténascine C. La stromélysine-1 active également les procollagénases (MMPs-1, -8 et -13) et la progélatinase B (MMP-9). La MMP-3 est incapable de dégrader les collagènes de type I et II, ce qui permet de la différencier de la MMP-1 (les deux enzymes ayant des masses moléculaires apparentes similaires). La MMP-3 est synthétisée sous la forme d'une préproenzyme avec un prépeptide de 17 acides aminés, un prodomaine de 82 acides aminés, un domaine catalytique de 165 acides aminés, une région charnière de 25 acides aminés et un domaine C-terminal de 188 acides aminés. La masse moléculaire apparente de la proenzyme est de 57 kDa et celle de la forme active est de 48 kDa. La MMP-3 est faiblement distribuée et peu exprimée par les cellules en culture.

Les glycolipides sont les dérivés glycosidiques des lipides tels que les acylglycérols (ou glycérides), les céramides ou les stérols. Ce groupe de composés peut d'ailleurs être considéré comme faisant partie d'une plus grande famille de composés, connue sous le terme générique de glycoconjugués. Le terme générique de glycosylglycérolipides est employé pour des composés glycolipidiques, renfermant une ou plusieurs unités de glycérol.
Les glycoglycérolipides sont pour la plupart amphiphiles, c'est-à-dire qu'ils possèdent une tête hydrophile et/ou polaire (sucres), et une tête hydrophobe et/ou apolaire (acide gras). Les acides gras composant ces glycolipides sont relativement variés (palmitique, stéarique, oléique, linoléique, linolénique ...). Les chaînes grasses insaturées sont essentiellement en C18, avec un nombre en insaturations allant de 1 à 3, et en C16, avec un nombre d'insaturations égal à 1 ou 2.

Le principal monosaccharride retrouvé dans les céréales, dont le blé, est le galactose. Il n'est pas inédit cependant de rencontrer dans d'autres plantes ou organites (cyanobactéries, champignons ...) d'autres structures de monosaccharides ou dérivés (glucose, rhamnose, N-acetylgalactosamine ...) et il est possible de trouver d'autres dérivés de monosaccharides tels que le xylose, l'arabinose, les acides uroniques comme l'acide galacturonique et l'acide glucuronique.

Les mono- et di-galactosyldiacylglycérols . (MGDG et DGDG) parmi les glycoglycérolipides, sont les principaux composants glycolipidiques des membranes biologiques végétales. Ce sont les lipides les plus abondants retrouvés dans la plupart des tissus photosynthétiques y compris les végétaux supérieurs, les algues et certaines bactéries. Dans les organes non-photosynthétiques, cette proportion en glycosyldiacylglycérols est cependant fortement réduite.

Les plus importantes sources de galactolipides sont les céréales et les grains. Les structures prédominantes, notamment dans le blé, sont les suivantes : 1,2-di-O-acyl-3-O-β- D-galactopyranosyl-*sn* -glycérol et 1,2-di-*O*-acyl-3-*O*-(6'-*O*-α-D-galactopyranosyl-β-Dgalactopyranosyl)-*sn*-glycérol, de formules suivantes :

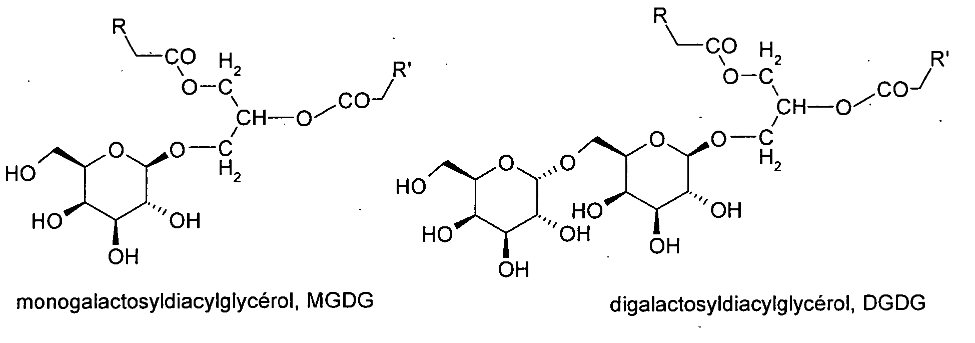

Les autres glycérides (MAG, MGMG, DGMG) sont mentionnés comme étant en proportion moindre dans les plantes telles que les céréales.

La présente invention a pour but de fournir de nouvelles compositions cosmétiques permettant d'inhiber partiellement les effets des radiations solaires UV-A impliquées dans le vieillissement cutané, lesdites compositions contenant des glycérides, glycosylés ou non, acylés ou non par des chaînes grâces insaturées ou non.

La présente invention a pour but de fournir de nouvelles compositions cosmétiques contenant des composés avec une activité d'inhibition directe des métalloprotéinases matricielles.

La présente invention concerne l'utilisation d'au moins un composé répondant à la formule (I) suivante : 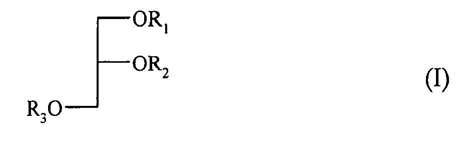 dans laquelle :
- R₁ représente un atome d'hydrogène ou un groupe acyle, saturé ou non, comprenant de 2 à 20 atomes de carbone,
- R₂ représente un atome d'hydrogène, un groupe acyle, saturé ou non, comprenant de 2 à 20 atomes de carbone, ou un groupe de formule suivante (A): 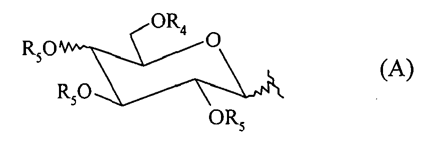 dans laquelle :
   * R₄ représente un atome d'hydrogène, un groupe acyle, saturé ou non, comprenant de 2 à 20 atomes de carbone, ou un groupe de formule (B) suivante : 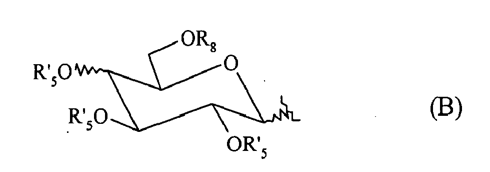
      - R'₅ représentant un atome d'hydrogène ou un groupe acyle, saturé ou non, comprenant de 2 à 20 atomes de carbone, et
      - R₈ représentant un atome d'hydrogène ou un groupe acyle, saturé ou non, comprenant de 2 à 20 atomes de carbone,
   * R₅ représente un atome d'hydrogène ou un groupe acyle, saturé ou non, comprenant de 2 à 20 atomes de carbone,
- R₃ représente un atome d'hydrogène ou un groupe de formule suivante (C) : 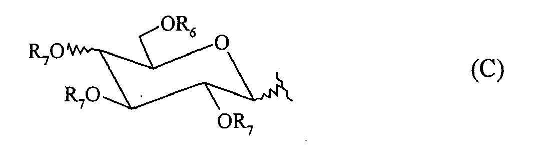 dans laquelle :
   * R₆ représente un atome d'hydrogène, un groupe acyle, saturé ou non, comprenant de 2 à 20 atomes de carbone, ou un groupe de formule (B) telle que définie ci-dessus,
   * R₇ représente un atome d'hydrogène ou un groupe acyle, saturé ou non, comprenant de 2 à 20 atomes de carbone,
pour la préparation d'une composition cosmétique, d'une composition pharmaceutique destinée au traitement des cancers n'impliquant pas le virus EBV, notamment destinée au traitement des mélanomes, ou destinée au traitement des processus inflammatoires de la sphère buccale, ou d'une composition nutraceutique,
- sous réserve que lorsque ledit composé est un composé de la famille des di-galactosyl-di-glycérides répondant à la formule (1) suivante : 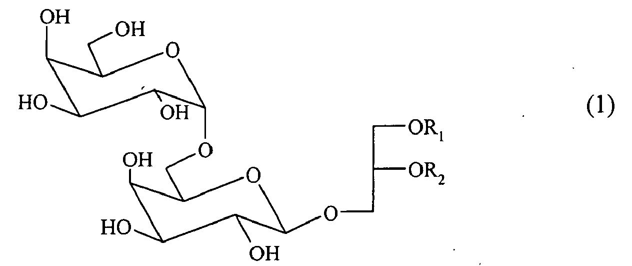 ladite composition cosmétique, pharmaceutique ou nutraceutique contient au moins un deuxième composé de formule (I) différent de celui de formule (1),
- et sous réserve que, dans la formule (I), lorsque R₁ et R₂ représentent un atome d'hydrogène, R₃ représente un groupe de formule (C),
- et sous réserve que, dans la formule (I), lorsque R₁ et R₂ représentent un groupe acyle, R₃ représente un groupe de formule (C),
- et sous réserve que les composés de formule (I) sont présents dans ladite composition cosmétique, pharmaceutique ou nutraceutique à des doses telles qu'ils ne présentent pas d'activité sur la synthèse de fibronectine, notamment déterminée par technique ELISA (QuantiMatrix Human Fibronectin ELISA kit, Références ECM300 Chemicon International).

Selon un mode de réalisation avantageux, l'utilisation susmentionnée est caractérisée en ce que :
- lorsque le composé est un composé de la famille des mono-galactosyl-di-glycérides répondant à la formule (2) suivante : 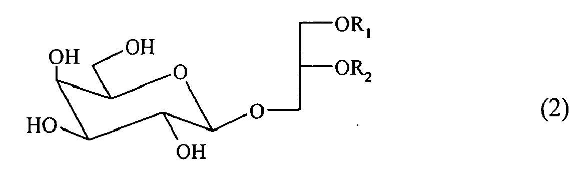 R₁ et R₂ étant tels que définis ci-dessus,
   ladite composition cosmétique, pharmaceutique ou nutraceutique contient au moins un deuxième composé de formule (I) différent de celui de formule (2),
- lorsque le composé est un composé de la famille des mono-galactosyl-mono-glycérides répondant à la formule (3) suivante : 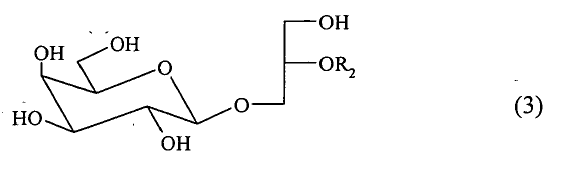 R₂ étant tel que défini ci-dessus,
   ladite composition cosmétique, pharmaceutique ou nutraceutique contient au moins un deuxième composé de formule (I) différent de celui de formule (3),
- lorsque le composé est un composé de la famille des mono-galactosyl-mono-glycérides répondant à la formule (4) suivante : 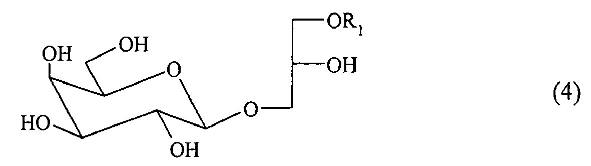 R₁ étant tel que défini ci-dessus,
   ladite composition cosmétique, pharmaceutique ou nutraceutique contient au moins un deuxième composé de formule (I) différent de celui de formule (4).

Les composés de formule (I) sont présents dans ladite composition cosmétique, pharmaceutique ou nutraceutique à des doses variant d'environ 0,01 à 1 mg/ml, et de préférence d'environ 0,05 à 0,5 mg/ml.

Le virus Epstein-Barr (EBV) est un virus ubiquitaire oncogène infectant la majorité de la population. Il est responsable en particulier de la mononucléose infectieuse. Ce principal syndrome clinique accompagne classiquement la primo-infection chez l'adolescent et l'adulte jeune. Mais ce virus est également associé au développement de différents cancers, notamment le cancer du rhinopharinx, le cancer du sein, le lymphome de Burkitt ou encore la maladie de Hodgkin.

Les composés de l'invention pourront être utilisés dans le traitement de l'ensemble des cancers et développements tumoraux n'impliquant pas le virus oncogène EBV. C'est le cas notamment des mélanomes et de la majorité des carcinomes cutanés, notamment des carcinomes squirrheux. Les composés de l'invention peuvent également être utilisés pour le traitement des fibrosarcomes.

Le composé (1) susmentionné, de la famille des di-galactosyl-di-glycérides (DGDG), correspond à un composé de formule (I) dans laquelle R₁ et R₂ représentent des groupes acyles et R₃ représente un groupe de formule (C) où R₆ représente un groupe galactosyle.

Les composés de formule (I) tels que définis ci-dessus sont des substances actives qui présentent une activité d'inhibition des métalloprotéinases.

Les composés de formule (I) tels que définis ci-dessus sont des substances actives qui ne présentent pas d'activité sur la fibronectine.

La synthèse de fibronectine en réponse aux composés de l'invention a été analysée par une technique de type ELISA (QuantiMatrix Human Fibronectin ELISA kit, Références ECM300 Chemicon International) et il a été constaté que ceux-ci ne présentent pas d'activité significative sur la synthèse de fibronectine intra- et extracellulaire, et ce jusqu'à des concentrations de 2 mg/ml.

Les compositions cosmétiques de l'invention ne contiennent pas de glycérol, correspondant à un composé de formule (I), dans laquelle R₁, R₂ et R₃ représentent un atome d'hydrogène, en tant que substance active. Si ces compositions cosmétiques contiennent du glycérol en tant qu'excipient, celui-ci ne présente pas d'activité d'inhibition des métalloprotéinases.

Les compositions cosmétiques de l'invention ne contiennent pas de diacétylglycérides (DAG), correspondant à un composé de formule (I), dans laquelle R₁ et R₂ représentent un groupe acyle et R₃ représente un atome d'hydrogène, en tant que substance active. Si ces compositions cosmétiques contiennent des DAG en tant qu'excipient, ceux-ci ne présentent pas d'activité d'inhibition des métalloprotéinases.

La présente invention a pour but de fournir des composés qui inhibent les métalloprotéinases *via* une action directe sur celles-ci, ce qui permet alors de rééquilibrer la balance entre dégradation et synthèse de la matrice extracellulaire dermique, et ainsi de limiter la dégradation. En effet, les MMP sont les principales enzymes impliquées dans cette dégradation lors des vieillissements cutanés ou lors du développement tumoral.

La présente invention a pour but de fournir des composés pouvant inhiber de façon sélective certaines de ces métalloprotéinases matricielles. En effet, ils inhibent de façon remarquable la collagénase de type I (MMP-1) sans pour autant avoir d'action sur l'activité des gélatinases. Ceci permet alors de limiter et de cibler l'action de la présente invention à l'inhibition de la dégradation de la matrice dermique associée aux vieillissements cutanés. En particulier, la présente invention permet de ne pas inhiber le phénomène de néo-angiogenèse (formation de nouveaux vaisseaux sanguins) particulièrement important lors de la cicatrisation cutanée et attribué principalement à la gélatinase A ou MMP-2 ainsi qu'à la MT1-MMP.

Ainsi, l'inhibition de l'activité de la plasmine, qui est à l'origine des cascades protéolytiques impliquées dans le vieillissement cutané, entraîne une inhibition de l'activation de la MMP-3 (stromélysine 1) mais également de la MMP-1 (collagénase 1), qui sont des enzymes particulièrement impliquées dans la dégradation de la matrice extracellulaire dermique.

La présente invention concerne également une composition nutraceutique contenant au moins un composé de formule (I) suivante : 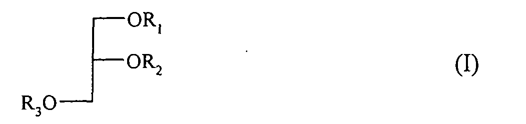 dans laquelle :
- R₁ représente un atome d'hydrogène ou un groupe acyle, saturé ou non, comprenant de 2 à 20 atomes de carbone,
- R₂ représente un atome d'hydrogène, un groupe acyle, saturé ou non, comprenant de 2 à 20 atomes de carbone, ou un groupe de formule suivante (A) : 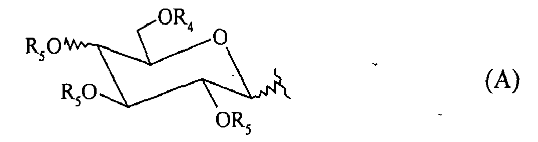 dans laquelle :
   * R₄ représente un atome d'hydrogène, un groupe acyle, saturé ou non, comprenant de 2 à 20 atomes de carbone, ou un groupe de formule (B) suivante : 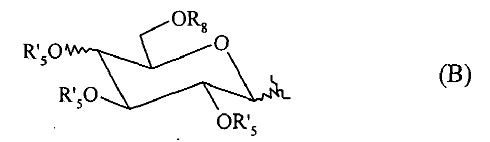
      - R'₅ représentant un atome d'hydrogène ou un groupe acyle, saturé ou non, comprenant de 2 à 20 atomes de carbone, et
      - R₈ représentant un atome d'hydrogène ou un groupe acyle, saturé ou non, comprenant de 2 à 20 atomes de carbone,
   * R₅ représente un atome d'hydrogène ou un groupe acyle, saturé ou non, comprenant de 2 à 20 atomes de carbone,
- R₃ représente un atome d'hydrogène ou un groupe de formule suivante (C) : 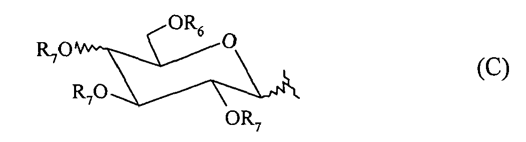 dans laquelle :
   * R₆ représente un atome d'hydrogène, un groupe acyle, saturé ou non, comprenant de 2 à 20 atomes de carbone, ou un groupe de formule (B) telle que définie ci-dessus,
   * R₇ représente un atome d'hydrogène ou un groupe acyle, saturé ou non, comprenant de 2 à 20 atomes de carbone,

- sous réserve que lorsque ledit composé est un composé de la famille des di-galactosyl-di-glycérides répondant à la formule (1) suivante : 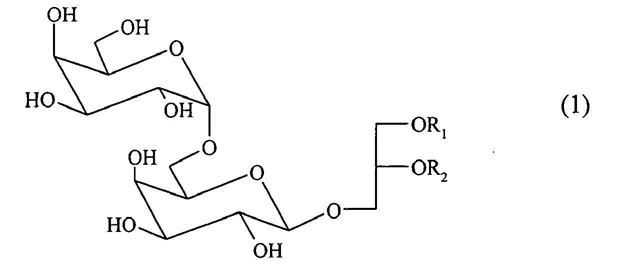 ladite composition nutraceutique contient au moins un deuxième composé de formule (I) différent de celui de formule (1),
- et sous réserve que, dans la formule (I), lorsque R₁ et R₂ représentent un atome d'hydrogène, R₃ représente un groupe de formule (C),
- et sous réserve que, dans la formule (I), lorsque R₁ et R₂ représentent un groupe acyle, R₃ représente un groupe de formule (C),
- et sous réserve que les composés de formule (I) sont présents dans ladite composition nutraceutique à des doses telles qu'ils ne présentent pas d'activité sur la fibronectine,
en association avec un véhicule approprié.

L'expression "véhicule approprié" désigne tout excipient, véhicule ou support cosmétiquement ou pharmaceutiquement, notamment dermatologiquement, acceptable. De tels supports sont bien connus de l'homme de l'art et résultent également de compositions cosmétiques, pharmaceutiques ou dermatologiques qui ont une portée générale. En exemple, les mélanges, les formules, les encapsulations monolamélaires (micelles), bilamellaires (liposomes), multilamellaires (sphérulites), et tout autre support connus en cosmétique et pharmaceutique peuvent être employés.

La présente invention concerne également une composition cosmétique contenant au moins un composé de formule (I) suivante : 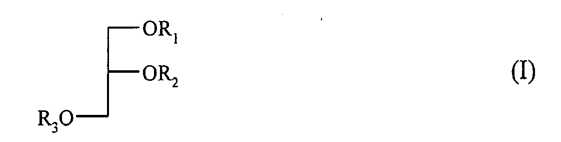 dans laquelle :
- R₁ représente un atome d'hydrogène ou un groupe acyle, saturé ou non, comprenant de 2 à 20 atomes de carbone,
- R₂ représente un atome d'hydrogène, un groupe acyle, saturé ou non, comprenant de 2 à 20 atomes de carbone, ou un groupe de formule suivante (A) : 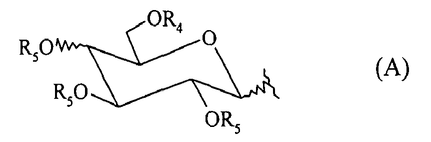 dans laquelle :
   * R₄ représente un atome d'hydrogène, un groupe acyle, saturé ou non, comprenant de 2 à 20 atomes de carbone, ou un groupe de formule (B) suivante : 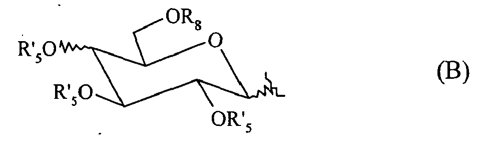
      - R'₅ représentant un atome d'hydrogène ou un groupe acyle, saturé ou non, comprenant de 2 à 20 atomes de carbone, et
      - R₈ représentant un atome d'hydrogène ou un groupe acyle, saturé ou non, comprenant de 2 à 20 atomes de carbone,
   * R₅ représente un atome d'hydrogène ou un groupe acyle, saturé ou non, comprenant de 2 à 20 atomes de carbone,
- R₃ représente un atome d'hydrogène ou un groupe de formule suivante (C) : 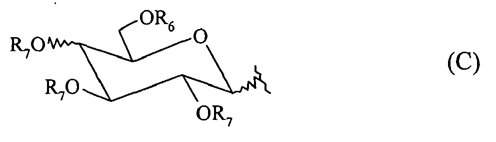 dans laquelle :
   * R₆ représente un atome d'hydrogène, un groupe acyle, saturé ou non, comprenant de 2 à 20 atomes de carbone, ou un groupe de formule (B) telle que définie ci-dessus,
   * R₇ représente un atome d'hydrogène ou un groupe acyle, saturé ou non, comprenant de 2 à 20 atomes de carbone,

- sous réserve que lorsque ledit composé est un composé de la famille des di-galactosyl-di-glycérides répondant à la formule (1) suivante : 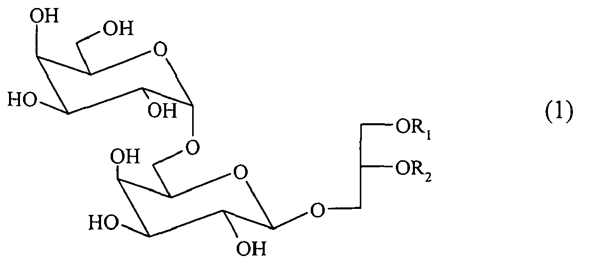 ladite composition cosmétique contient au moins un deuxième composé de formule (I) différent de celui de formule (1),
- et sous réserve que, dans la formule (I), lorsque R₁ et R₂ représentent un atome d'hydrogène, R₃ représente un groupe de formule (C),
- et sous réserve que, dans la formule (I), lorsque R₁ et R₂ représentent un groupe acyle, R₃ représente un groupe de formule (C),
- et sous réserve que les composés de formule (I) sont présents dans ladite composition cosmétique à des doses telles qu'ils ne présentent pas d'activité sur la fibronectine, notamment dans un test de mesure par technique de type ELISA (QuantiMatrix Human Fibronectin ELISA kit, Références ECM300 Chemicon International),
en association avec un véhicule approprié.

Selon un mode de réalisation avantageux, la présente invention concerne une composition cosmétique telle que définie ci-dessus, caractérisée en ce que :
- lorsque le composé est un composé de la famille des mono-galactosyl-di-glycérides répondant à la formule (2) suivante : 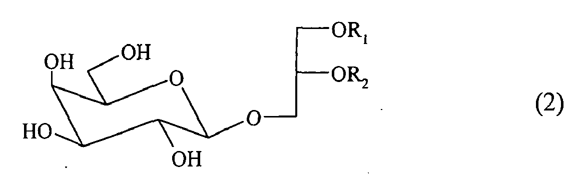 R₁ et R₂ étant tels que définis ci-dessus,
   ladite composition cosmétique contient au moins un deuxième composé de formule (I) différent de celui de formule (2),
- lorsque le composé est un composé de la famille des mono-galactosyl-mono-glycérides répondant à la formule (3) suivante : 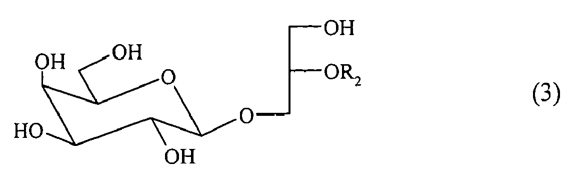 R₂ étant tel que défini ci-dessus,
   ladite composition cosmétique contient au moins un deuxième composé de formule (I) différent de celui de formule (3),
- lorsque le composé est un composé de la famille des mono-galactosyl-mono-glycérides répondant à la formule (4) suivante : 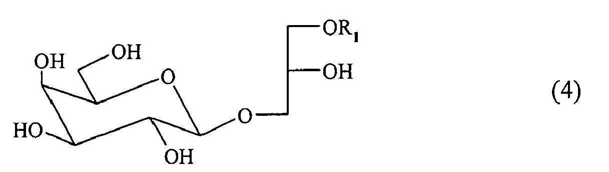 R₁ étant tel que défini ci-dessus,
   ladite composition cosmétique contient au moins un deuxième composé de formule (I) différent de celui de formule (4).

L'expression "véhicule approprié" désigne tout excipient, véhicule ou support acceptable d'un point de vue cosmétique ou pharmaceutique, notamment dermatologique. De tels supports sont bien connus de l'homme de l'art et résultent également de compositions cosmétiques, pharmaceutiques ou dermatologiques qui ont une portée générale. En exemple, les mélanges, les formules, les encapsulations monolamélaires (micelles), bilamellaires (liposomes), multilamellaires (sphérulites), et tout autre support connus en cosmétique et pharmaceutique peuvent être employés.

Selon un mode de réalisation avantageux, la composition cosmétique selon la présente invention est caractérisée en ce qu'elle contient au moins un composé de formule (I) dans laquelle R₃ représente un atome d'hydrogène.

Ainsi, une telle composition cosmétique contient des composés répondant à la formule (II) suivante : 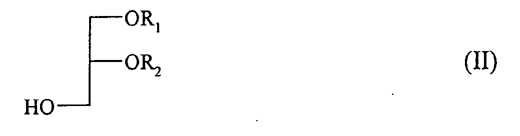
R₁ représentant un atome d'hydrogène ou un groupe acyle, saturé ou non, comprenant de 2 à 20 atomes de carbone,
R₂ représentant un atome d'hydrogène, un groupe acyle, saturé ou non, comprenant de 2 à 20 atomes de carbone, ou un groupe de formule (A) telle que définie ci-dessus,
sous réserve que lorsque R₁ représente un groupe acyle, R₂ représente un atome d'hydrogène ou un groupe de formule (A) telle que définie ci-dessus, et
sous réserve que lorsque R₁ représente un atome d'hydrogène, R₂ représente un groupe acyle ou un groupe de formule (A).

Une composition préférée selon la présente invention contient au moins un composé répondant à la formule (II) susmentionnée, dans laquelle :
- soit R₁ représente un atome d'hydrogène et R₂ représente un groupe acyle, saturé ou non, comprenant de 2 à 20 atomes de carbone,
- soit R₁ représente un groupe acyle, saturé ou non, comprenant de 2 à 20 atomes de carbone et R₂ représente un atome d'hydrogène.

Ce mode de réalisation correspond à des compositions cosmétiques contenant au moins un composé qui ne comprend pas de sucre, tels que des composés de la famille des mono-acétyl-glycérides (MAG).

Selon un mode de réalisation avantageux, la composition cosmétique selon la présente invention est caractérisée en ce qu'elle contient au moins un composé de formule (I) dans laquelle R₃ représente un groupe de formule (C) telle que définie ci-dessus.

Ainsi, une telle composition cosmétique contient au moins un composé répondant à la formule (I-4) suivante : 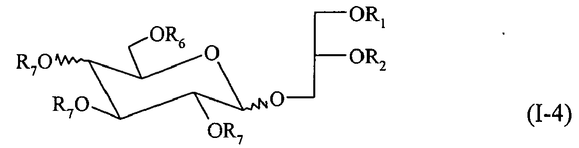 R₁, R₂, R₆ et R₇ étant tels que définis ci-dessus.

Ce mode de réalisation correspond à des compositions cosmétiques contenant au moins un composé qui comprend au moins un sucre.

Selon un mode de réalisation avantageux, la composition cosmétique selon la présente invention est caractérisée en ce qu'elle contient au moins un composé de formule (I-4) telle que définie ci-dessus, dans laquelle R₆ représente un atome d'hydrogène.

Ainsi, une telle composition cosmétique contient au moins un composé répondant à la formule (I-3) suivante : 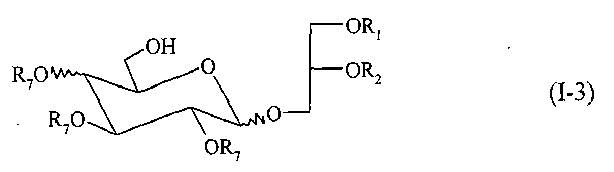 R₁, R₂ et R₇ étant tels que définis ci-dessus.

Une composition préférée selon la présente invention contient au moins un composé répondant à la formule (I-3) susmentionnée, dans laquelle :
R₁ représente un atome d'hydrogène ou un groupe acyle, saturé ou non, comprenant de 2 à 20 atomes de carbone, et
R₂ représente un groupe acyle, saturé ou non, comprenant de 2 à 20 atomes de carbone ou un atome d'hydrogène.

Ce mode de réalisation préféré correspond à des compositions cosmétiques contenant au moins un composé qui comprend un seul sucre non acylé, tels que des composés de la famille des mono-galactosyl-mono-glycérides (MGMG) ou de la famille des mono-galactosyl-di-glycérides (MGDG).

Les composés de la famille des MGMG sont des composés de formule (I-4) susmentionnée dans laquelle R₁ ou R₂ représente un groupe acyle.

Les composés de la famille des MGDG sont des composés de formule (I-4) susmentionnée dans laquelle R₁ et R₂ représentent un groupe acyle.

Selon un mode de réalisation avantageux, la composition cosmétique de l'invention est caractérisée en ce qu'elle contient au moins un composé de formule (1-4) telle que définie ci-dessus, dans laquelle R₆ représente un groupe acyle tel que défini ci-dessus.

Ce mode de réalisation préféré correspond à des compositions cosmétiques contenant au moins un composé qui comprend un sucre acylé, tels que des composés de la famille des mono-acétyl-mono-galactosyl-di-glycérides (MAMGDG).

Selon un mode de réalisation avantageux, la composition cosmétique de l'invention est caractérisée en ce qu'elle contient au moins un composé de formule (I-4) telle que définie ci-dessus, dans laquelle R₆ représente un groupe de formule (B) telle que définie ci-dessus.

Ainsi, une telle composition cosmétique contient au moins un composé répondant à la formule (I-6) suivante : 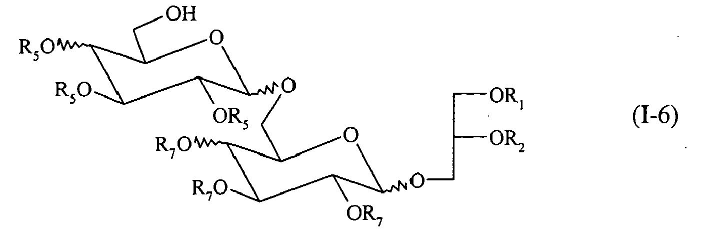 R₁, R₂, R₅ et R₇ étant tels que définis ci-dessus.

Ce mode de réalisation avantageux correspond à des compositions cosmétiques contenant au moins un composé qui comprend un sucre substitué par un autre sucre, tels que des composés de la famille des di-galactosyl-mono-glycérides (DGMG) ou de la famille des di-galactosyl-di-glycérides (DGDG).

Les composés de la famille des DGMG sont des composés de formule (I-6) susmentionnée dans laquelle R₁ ou R₂ représente un groupe acyle.

Les composés de la famille des DGDG sont des composés de formule (I-6) susmentionnée dans laquelle R₁ et R₂ représentent un groupe acyle.

Selon un mode de réalisation avantageux, la composition cosmétique de l'invention est caractérisée en ce qu'elle contient au moins un composé de formule (I) dans laquelle l'un au moins des groupes R₁ et R₂ représente un groupe acyle tel que défini ci-dessus.

Selon un mode de réalisation avantageux, la composition cosmétique de l'invention est caractérisée en ce qu'elle contient au moins un composé de formule (I) dans laquelle R₁ et R₂ représentent un groupe acyle tel que défini ci-dessus.

Une autre composition cosmétique avantageuse de la présente invention est caractérisée en ce qu'elle contient au moins un composé répondant à la formule (II-bis) suivante : 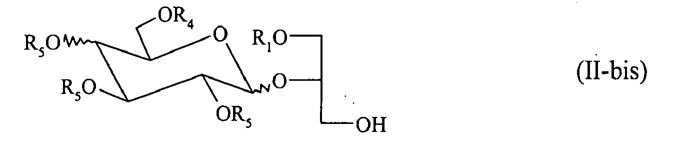 R₁, R₄ et R₅ étant tels que définis ci-dessus.

Une composition cosmétique avantageuse de l'invention est caractérisée en ce qu'elle comprend au moins un composé de formule (I) dans laquelle :
- R₁ représente un groupe acyle, saturé ou non, comprenant de 2 à 20 atomes de carbone, et représente de préférence un groupe linoléyle, palmitoléyle, palmityle ou stéaryle, ou R₁ représente un atome d'hydrogène,
- R₂ représente un atome d'hydrogène lorsque R₁ représente un groupe acyle tel que défini ci-dessus, ou R₂ représente un groupe acyle, saturé ou non, comprenant de 2 à 20 atomes de carbone, et de préférence un groupe linoléyle, palmitoléyle, palmityle ou stéaryle, lorsque R₁ représente un atome d'hydrogène, et
- R₃ représente un atome d'hydrogène.

Un tel composé répond à l'une des formules (I-1) ou (I-1') suivantes :

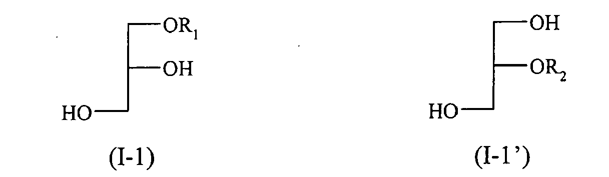

De tels composés appartiennent à la famille des mono-acétyl-glycérides (MAG).

Un composé préféré de formule (I-1) est un composé dans lequel R₁ représente un groupe linoléyle. Un tel composé répond à la formule suivante : 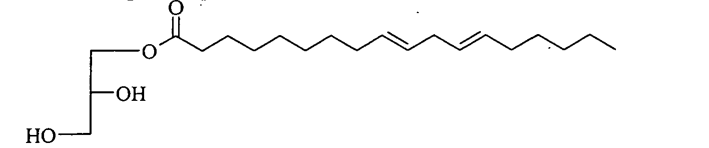 Ce composé est le 1-O-linoléyl-*sn*-glycérol.

Une autre composition cosmétique avantageuse de l'invention est caractérisée en ce qu'elle comprend au moins un composé de formule (I) dans laquelle :
- R₁ représente un atome d'hydrogène ou un groupe acyle, saturé ou non, comprenant de 2 à 20 atomes de carbone, de préférence un groupe linoléyle, palmitoléyle, palmityle ou stéaryle,
- R₂ représente un atome d'hydrogène lorsque R₁ représente un groupe acyle tel que défini ci-dessus, ou R₂ représente un groupe acyle, saturé ou non, comprenant de 2 à 20 atomes de carbone, de préférence un groupe linoléyle, palmitoléyle, palmityle ou stéaryle, lorsque R₁ représente un atome d'hydrogène,
- R₃ représente un groupe de formule (C) telle que définie ci-dessus, dans laquelle R₆ représente un atome d'hydrogène.

Un tel composé répond à l'une des formules (I-2) ou (I-2') suivantes :

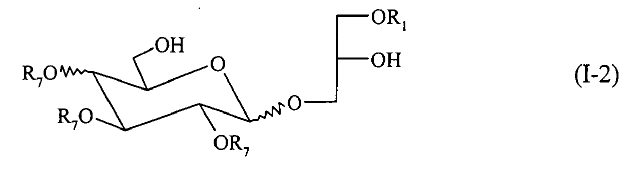

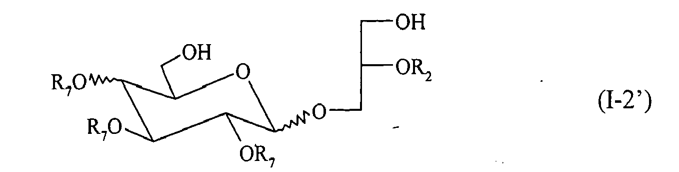

Selon un autre mode de réalisation avantageux, la composition cosmétique de l'invention contient au moins un composé répondant à l'une des formules (I-2-bis), (I-2-ter), (I-2'-bis) ou (I-2'-ter) suivantes :

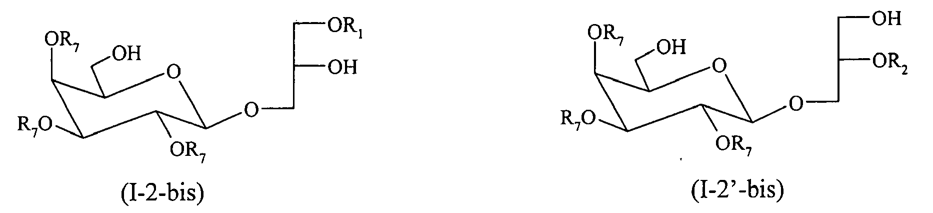

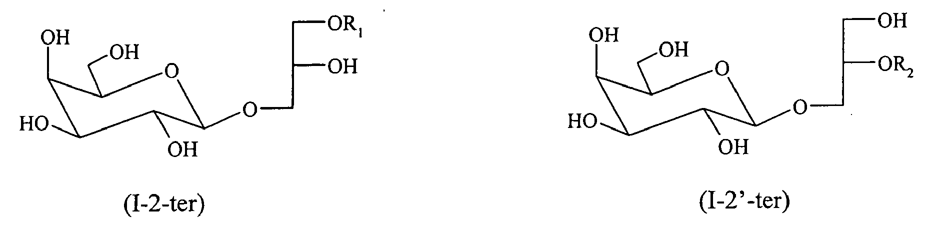

Les composés de formule (I-2-ter) correspondent aux composés de formule (I-2-bis) dans laquelle R₇ représente un atome d'hydrogène.

Les composés de formule (I-2'-ter) correspondent aux composés de formule (I-2'-bis) dans laquelle R₇ représente un atome d'hydrogène.

De tels composés appartiennent à la famille des mono-galactosyl-mono-glycérides (MGMG).

Un composé préféré de formule (I-2) est un composé de formule (I-2-ter) dans laquelle R₁ représente un groupe linoléyle. Un tel composé répond à la formule suivante :

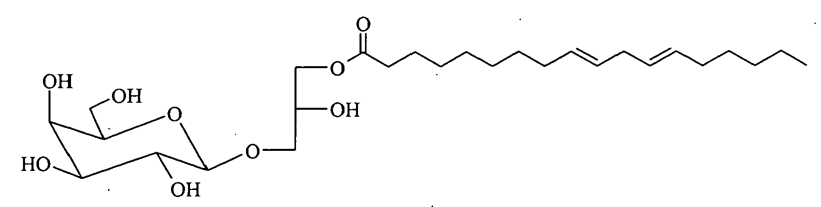

Ce composé est le 1-O-linoléyl-3-O-β-D-galactopyranosyl-*sn*-glycérol.

Une autre composition cosmétique avantageuse de l'invention est caractérisée en ce qu'elle comprend au moins un composé de formule (I) dans laquelle :
- R₁ représente un groupe acyle, saturé ou non, comprenant de 2 à 20 atomes de carbone, et de préférence un groupe linoléyle,
- R₂ représente un groupe acyle, saturé ou non, comprenant de 2 à 20 atomes de carbone, et de préférence un groupe linoléyle, et
- R₃ représente un groupe de formule (C) telle que définie ci-dessus, dans laquelle R₆ représente un atome d'hydrogène.

Un tel composé répond à la formule (I-3) suivante :

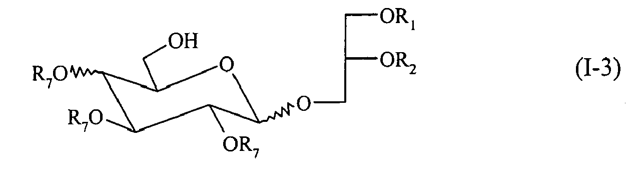

Selon un autre mode de réalisation avantageux, la composition cosmétique de l'invention contient au moins un composé répondant à l'une des formules (I-3-bis) ou (I-3-ter) suivantes :

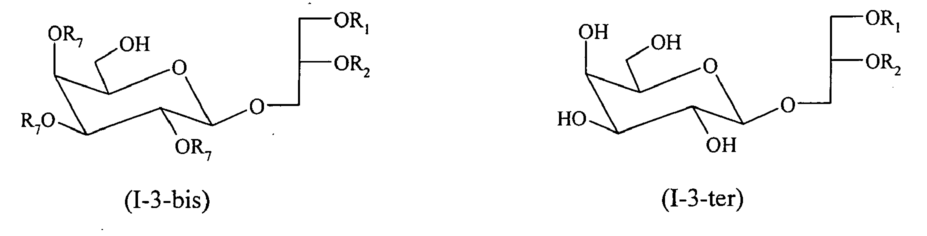

Les composés de formule (I-3-ter) correspondent aux composés de formule (I-3-bis) dans laquelle R₇ représente un atome d'hydrogène.

De tels composés appartiennent à la famille des mono-galactosyl-di-glycérides (MGDG).

Un composé préféré de formule (I-3) est un composé de formule (I-3-ter) dans laquelle R₁ représente un groupe linoléyle et R₂ représente un groupe palmityle. Un tel composé répond à la formule suivante :

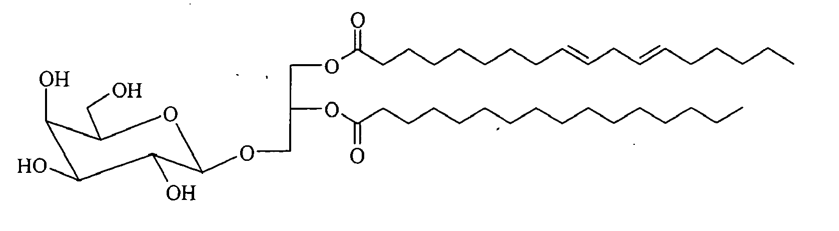

Ce composé est le 1-O-linoléyl-2-O-palmityl-3-O-β-D-galactopyranosyl-*sn-*glycérol.

Un autre composé préféré de formule (I-3) est un composé de formule (I-3-ter) dans laquelle R₁ et R₂ représentent un groupe linoléyle. Un tel composé répond à la formule suivante :

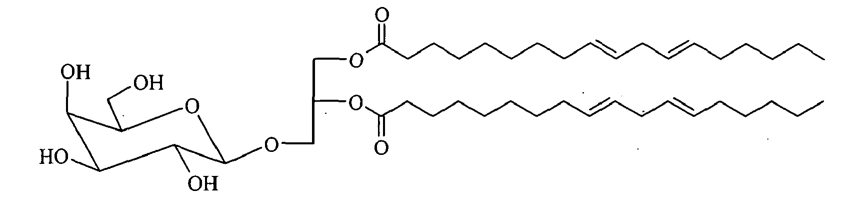

Ce composé est le 1,2-di-O-linoléyl-3-O-β-D-galactopyranosyl- *sn*-glycérol.

Une autre composition cosmétique avantageuse de l'invention est caractérisée en ce qu'elle comprend au moins un composé de formule (I) dans laquelle :
- R₁ et R₂ représentent un groupe acyle, saturé ou non, comprenant de 2 à 20 atomes de carbone, et de préférence un groupe linoléyle, et
- R₃ représente un groupe de formule (C) telle que définie dans la revendication 2, dans laquelle R₆ représente un groupe acyle, saturé ou non, comprenant de 2 à 20 atomes de carbone.

Un tel composé répond à la formule (I-4) suivante :

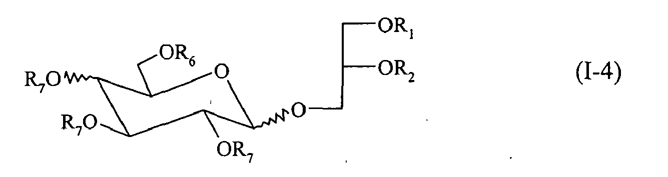

Selon un autre mode de réalisation avantageux, la composition cosmétique de l'invention contient au moins un composé répondant à l'une des formules (I-4-bis) ou (I-4-ter) suivantes :

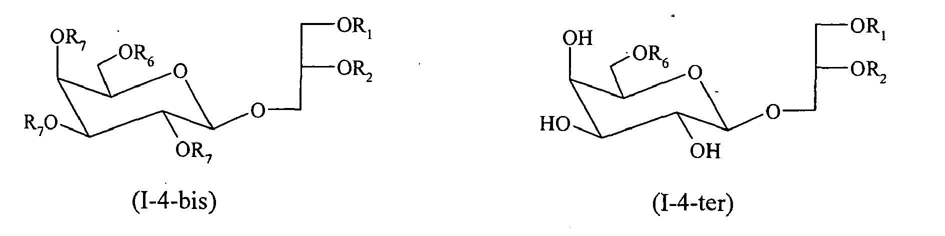

Les composés de formule (I-4-ter) correspondent aux composés de formule (I-4-bis) dans laquelle R₇ représente un atome d'hydrogène.

De tels composés appartiennent à la famille des mono-acétyl-mono-galactosyl-di-glycérides (MAMGDG).

Un composé préféré de formule (I-4) est un composé de formule (I-4-ter) dans laquelle R₁ et R₂ représentent un groupe linoléyle et R₆ représente un groupe palmitoyle. Un tel composé répond à la formule suivante :

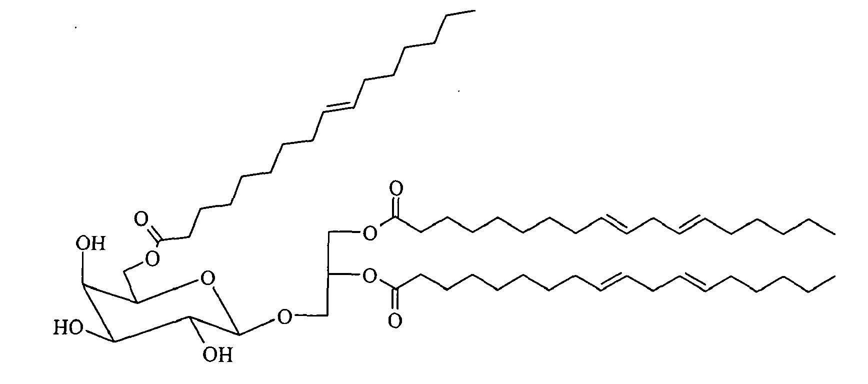

Ce composé est le 1,2-di-O-linoléyl-3-O-[6'-O-palmitoyl-β-D-galactopyranosyl]-*sn*-glycérol (ED23-11).

Une autre composition cosmétique avantageuse de l'invention est caractérisée en ce qu'elle comprend au moins un composé de formule (I) dans laquelle :
- R₁ représente un atome d'hydrogène, ou R₁ représente un groupe acyle, saturé ou non, comprenant de 2 à 20 atomes de carbone, et de préférence un groupe linoléyle,
- R₂ représente un atome d'hydrogène lorsque R₁ représente un groupe acyle tel que défini ci-dessus, ou R₂ représente un groupe acyle, saturé ou non, comprenant de 2 à 20 atomes de carbone, et de préférence un groupe linoléyle, lorsque R₁ représente un atome d'hydrogène, et
- R₃ représente un groupe de formule (C) telle que définie ci-dessus, dans laquelle R₆ représente un groupe de formule (B) telle que définie ci-dessus.

Un tel composé répond à l'une des formules (I-5) ou (I-5') suivantes :

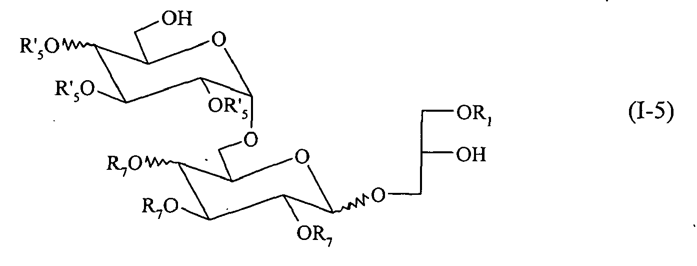

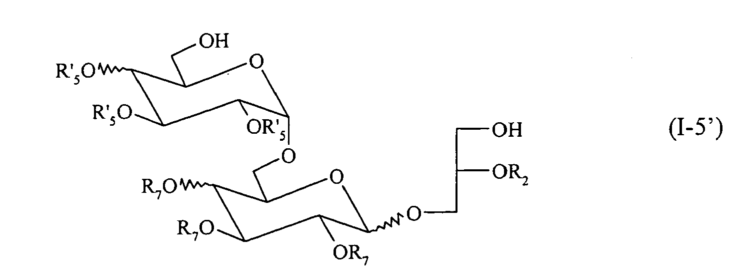

Selon un autre mode de réalisation avantageux, la composition cosmétique de l'invention contient au moins un composé répondant à l'une des formules (I-5-bis), (I-5-ter), (I-5'-bis) ou (I-5'-ter) suivantes :

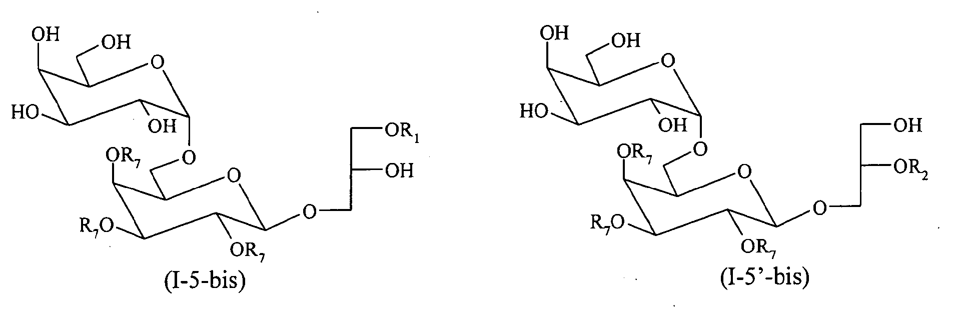

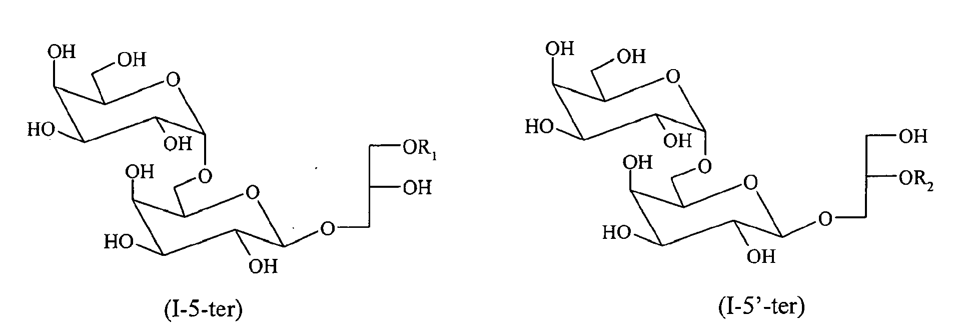

Les composés de formule (I-5-ter) correspondent aux composés de formule (I-5-bis) dans laquelle R₇ représente un atome d'hydrogène.

Les composés de formule (I-5'-ter) correspondent aux composés de formule (I-5'-bis) dans laquelle R₇ représente un atome d'hydrogène.

De tels composés appartiennent à la famille des di-galactosyl-mono-glycérides (DGMG).

Un composé préféré de formule (I-5) est un composé de formule (I-5-ter) dans laquelle R₁ représente un groupe linoléyle. Un tel composé répond à la formule suivante :

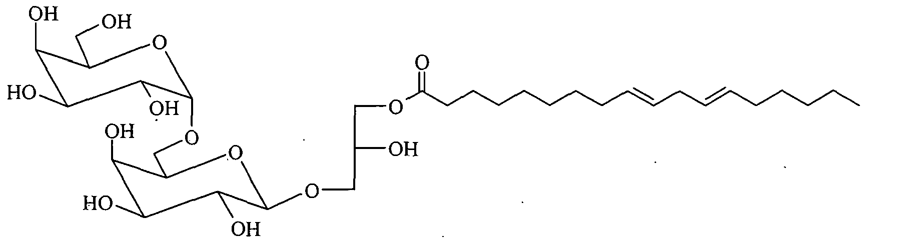

Ce composé est le 1-O-linoléyl-3-O-[6'-O-α-D-galactopyranosyl-β-D-galactopyranosyl] -sn-glycérol.

Une autre composition cosmétique avantageuse de l'invention est caractérisée en ce qu'elle comprend au moins un composé de formule (I) dans laquelle :
- R₁ représente un groupe acyle, saturé ou non, comprenant de 2 à 20 atomes de carbone, et de préférence un groupe linoléyle,
- R₂ représente un groupe acyle, saturé ou non, comprenant de 2 à 20 atomes de carbone, et de préférence un groupe linoléyle, et
- R₃ représente un groupe de formule (C) telle que définie ci-dessus, dans laquelle R₆ représente un groupe de formule (B) telle que définie ci-dessus.

Un tel composé répond à la formule (1-6) suivante :

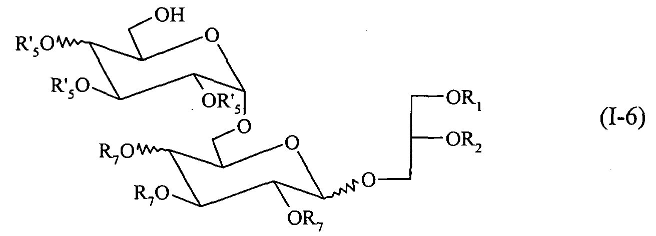

Selon un autre mode de réalisation avantageux, la composition cosmétique de l'invention contient au moins un composé répondant à l'une des formules (I-6-bis) ou (I-6-ter) suivantes :

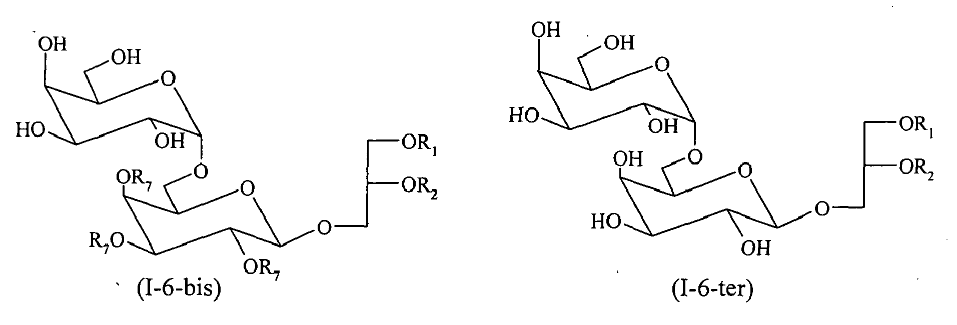

Les composés de formule (I-6-ter) correspondent aux composés de formule (I-6-bis) dans laquelle R₇ représente un atome d'hydrogène.

De tels composés appartiennent à la famille des di-galactosyl-di-glycérides (DGDG).

Un composé préféré de formule (I-6) est un composé de formule (I-6-ter) dans laquelle R₁ et R₂ représentent un groupe linoléyle. Un tel composé répond à la formule suivante :

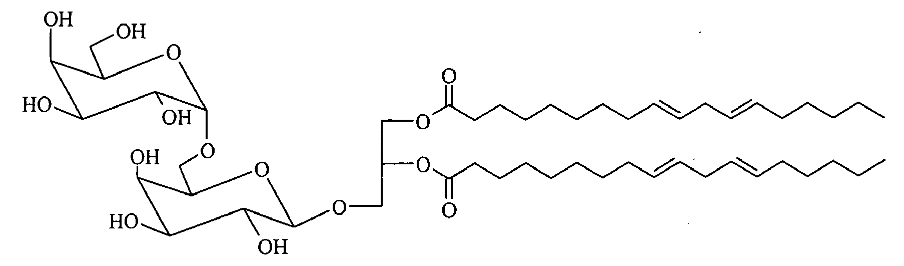

Ce composé est le 1,2-di-O-linoléyl-3-O-[6'-O-α-D-galactopyranosyl-β-D-galactopyranosyl]-*sn*-glycérol.

Un composé préféré de formule (I-6) est un composé de formule (I-6-ter) dans laquelle R₁ représente un groupe linoléyle et R₂ représente un groupe palmityle. Un tel composé répond à la formule suivante :

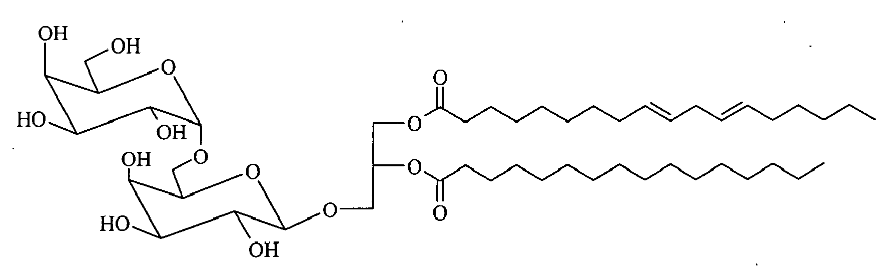

Ce composé est le 2-O-palmityl-3-O-linoléyl-1-O-[6'-O-α-D-galactopyranosyl-β-D-galactopyranosyl]-D-glycérine.

La présente invention concerne également une composition cosmétique telle que définie ci-dessus, caractérisée en ce qu'elle comprend au moins un composé de formule (I) dans laquelle R₁ et/ou R₂ représente(nt) un groupe acyle comprenant au moins une insaturation.

Parmi les groupes acyles comprenant au moins une insaturation, on peut citer les groupes palmitoyle, linoléyle ou linolényle.

La présente invention concerne également une composition cosmétique telle que définie ci-dessus, caractérisée en ce que les composés de formule (I) présentent une activité d'inhibition des métalloprotéinases matricielles, notamment d'inhibition des collagénases.

La mesure de l'activité enzymatique des MMPs est basée sur le principe du transfert d'énergie par résonance, RET ou transfert d'énergie de fluorescence par résonance, FRET. Le substrat est constitué d'un oligopeptide comportant un groupement fluorescent (F), donneur d'énergie et un groupement éteignant (Q, pour quenching), accepteur d'énergie. Après hydrolyse, le groupement éteignant est libéré, permettant de mesurer l'augmentation de fluorescence.

De nombreux couples fluorescent/éteignant ont été développés pour la mesure de l'activité enzymatique des MMPs comme le couple 7-méthoxycoumarine-4-acétyl (Mca)/dinitrophenyl-diaminopropionyl (Dnp).

Après activation, la MMP-1 est pré-incubée à +20°C pendant 10 minutes en absence ou en présence d'effecteurs dans un tampon Tris-HCl 50 mM, NaCl 150 mM, CaCl₂ 5 mM pH 7,5 en plaque 96 puits noire dont les sites de liaisons non-spécifiques ont été bloqués à l'aide d'une solution de SAB à 0,1% (p/v) dans le même tampon. La réaction est initiée par addition de 10 µL de substrat à la concentration finale de 2 µM dans un volume réactionnel final de 200 µL. Les variations de fluorescence (λ excitation : 360 nm, λ émission : 465 nm) au cours du temps sont suivies à l'aide d'un spectrofluorimètre lecteur de plaque HTS 7000+ à +20°C. La courbe représentant la fluorescence (en RFU) en fonction du temps (en minutes) est tracée.

Les intensités de fluorescence obtenues permettent d'estimer là vitesse initiale de l'enzyme en absence d'effecteur (V₀) et en présence d'inhibiteur (Vᵢ). Le rapport Vᵢ/V₀ est calculé pour chaque concentration de produit et permet de déterminer l'activité de l'enzyme en présence d'effecteur, exprimée en pourcentage de l'activité de l'enzyme seule.

La présente invention concerne également une composition cosmétique telle que définie ci-dessus, caractérisée en ce qu'elle contient des céramides.

La présente invention concerne également une composition cosmétique telle que définie ci-dessus, caractérisée en ce qu'elle ne contient pas de céramides.

Selon un mode de réalisation avantageux, la composition de l'invention est destinée à une application topique.

Selon un mode de réalisation avantageux, la composition de l'invention est utilisée en association avec un filtre solaire. Selon un autre mode de réalisation avantageux, la composition peut être utilisée en association avec les autres excipients, ingrédients ou actifs usuels et connus de l'homme de l'art en formulation cosmétique.

La présente invention concerne également une méthode de traitement cosmétique, comprenant l'administration d'une quantité appropriée d'une composition cosmétique telle que définie ci-dessus.

La méthode de traitement cosmétique selon l'invention permet ainsi de prévenir le vieillissement chronologique et photo-induit dû aux UVA du derme principalement au travers d'une action anti-collagénolytique.

L'exposition au soleil peut induire des effets rapides et profonds sur l'organisme. On parle de réactions aiguës. Elles englobent la réaction inflammatoire et les événements immunologiques qui en découlent. Les effets à long terme de ces expositions ont pour principale conséquence le vieillissement cutané. Le vieillissement photo-induit regroupe l'ensemble des phénomènes observés lors du vieillissement chronologique, mais ils sont exacerbés au niveau des zones de l'épiderme en contact avec l'environnement et plus particulièrement avec les ultraviolets.

Les rayons ultraviolets sont divisés en plusieurs types selon leurs longueurs d'onde : Ultraviolets C = de 200 à 280 nm ; Ultraviolets B = de 280 à 315 nm et Ultraviolets A = de 315 à 400 nm. La couche d'ozone contenue dans l'atmosphère offre une protection au rayonnement ultraviolet en filtrant les plus énergétiques, de longueur d'onde inférieure à 295 nm, c'est à dire les UVC et une partie des UVB, permettant ainsi le maintien de la vie sur terre. Les radiations lumineuses peuvent provoquer au niveau des tissus des réactions mécaniques, thermiques et photochimiques. Les dommages thermiques (photo-coagulation) sont provoqués, au niveau tissulaire, par une augmentation importante de la température et une coagulation des protéines. Les dommages photochimiques nécessitent la présence d'une radiation de longueur d'onde appropriée, d'une molécule absorbante ou chromophore, d'une durée et d'une dose d'irradiation suffisante.

Le vieillissement photo-induit est particulièrement important au niveau du visage et des membres. 80% du vieillissement du visage est attribué au rayonnement solaire (Gilchrest B.A., Skin aging and photoaging: an overview. J Am Acad Dermatol 1989; 21(3 Pt 2): 610-3). Il se caractérise par une modification de la matrice extracellulaire du derme, nommée élastose, une diminution de la couche épidermique et une augmentation non homogène de la pigmentation cutanée. Cliniquement, des rides, beaucoup plus profondes que dans le vieillissement chronologique, apparaissent (Kligman L.H. et Kligman A.M., The nature of photoaging: its prevention and repair. Photodermatol 1986; 3(4): 215-27).

Contrairement au réseau de fibres élastiques, l'expression des composants du réseau de collagène comme le collagène de type I et la décorine est diminuée dans la peau photo-vieillie (Bernstein E.F., Fisher L.W., Li K., Lebaron R.G., Tan E.M. et Uitto J., Differential expression of the versican and decorin genes in photoaged and sun-protected skin. Comparison by immunohistochemical and northern analyses. Lab Invest 1995; 72(6): 662-9 ; Varani J., Spearman D., Perone P., Fligiel S.E., Datta S.C., Wang Z.Q., et al., Inhibition of type I procollagen synthesis by damaged collagen in photoaged skin and by collagenase-degraded collagen in vitro. Am J Pathol 2001; 158(3): 931-42). Cette réduction est accompagnée d'une dégradation du réseau collagénique existant (Bernstein E.F. et Uitto J., The effect of photodamage on dermal extracellular matrix. Clin Dermatol 1996; 14(2): 143-51). Comme pour la dégradation des fibres élastiques, la dégradation du réseau de collagène est principalement due à l'augmentation de l'expression des MMPs (Griffiths C.E., Russman A.N., Majmudar G., Singer R.S., Hamilton T.A. et Voorhees J.J., Restoration of collagen formation in photodamaged human skin by tretinoin (retinoic acid). N Engl J Med 1993; 329(8): 530-5). En effet, l'accumulation de matrikines, fragments de collagènes et d'élastine possédant une activité biologique, entraîne l'augmentation de l'expression des MMPs (Huet E., Brassart B., Wallach J., Debelle L., Haye B., Emonard H. et Hornebeck W., Influence des peptides d'élastine sur la production de la métalloprotéinase matricielle-2 par les fibroblastes du derme humain en culture. J Soc Biol 2001; 195: 165-172), ainsi que la diminution de la prolifération et de la synthèse de collagène par les fibroblastes dermiques (Varani J., Spearman D., Perone P., Fligiel S.E., Datta S.C., Wang Z.Q., et al., Inhibition of type I procollagen synthesis by damaged collagen in photoaged skin and by collagenase-degraded collagen in vitro. Am J Pathol 2001; 158(3): 931-42). On note également une altération de la microvasculature dermique, les capillaires apparaissent raréfiés, dilatés, tortueux. Il existe un épaississement marqué de la paroi des veinules post-capillaires.

Les altérations des structures, fonctions, et en finalité apparence de la peau sont, comme dans le cas du vieillissement chronologique, principalement dues à la formation et à la libération de radicaux libres (RLO). Les UV sont absorbés au niveau de la peau par différents chromophores. L'énergie reçue entraîne de nombreuses réactions chimiques : d'une part l'absorption des UVB par l'ADN est responsable de liaisons croisées des bases pyrimidiques, d'autre part l'absorption des UVA par des chromophores cutanés entraîne un transfert d'énergie à l'oxygène aboutissant à la formation de RLO. Ils permettent alors l'oxydation de nombreux constituants cellulaires comme les protéines, les lipides et l'ADN. En 1998, Fisher et Voorhess ont décrit un modèle physiopathologique du vieillissement photo-induit. En favorisant la formation de RLO les radiations UV "activeraient" les récepteurs de facteurs de croissance et de cytokines à la surface cellulaire des kératinocytes et des fibroblastes. Cette activation des récepteurs peut également être induite par autophosphorylation sous l'effet des UV, comme dans le cas du récepteur à l'EGF (Fisher G.J. et Voorhees J.J., Molecular mechanisms of photoaging and its prevention by retinoic acid: ultraviolet irradiation induces MAP kinase signal transduction cascades that induce Ap-1-regulated matrix metalloproteinases that degrade human skin in vivo. J Investig Dermatol Symp Proc 1998; 3(1): 61-8). La stimulation des récepteurs conduit à la transduction du signal via les cascades des protéines kinases qui activent le facteur de transcription AP-1 au niveau du noyau des cellules. L'activation d'AP-1 stimule alors la transcription des gènes codant pour la MMP-1, la MMP-9 ainsi que la MMP-3 (Fisher G.J., Datta S.C., Talwar H.S., Wang Z.Q., Varani J., Kang S. et Voorhees J.J., Molecular basis of sun-induced premature skin ageing and retinoid antagonism. Nature 1996; 379(6563): 335-9). L'induction de ces MMPs à la fois dans le derme et l'épiderme favorise la dégradation des collagènes, de l'élastine, mais également de la membrane basale et des protéoglycanes (Kahari V.M. et Saarialho-Kere U., Matrix metalloproteinases in skin. Exp Dermatol 1997; 6(5): 199-213). Cette dégradation de la matrice dermique serait suivie d'une réparation imparfaite, conduisant à des défauts dans la structure et l'organisation de la matrice extracellulaire. Cette réparation imparfaite est appelée cicatrisation solaire.

La présente invention concerne également un procédé de préparation d'une composition cosmétique telle que définie ci-dessus et contenant des fractions lipidiques enrichies en composés de formule (I) ou des sous-fractions contenant au moins un composé de formule (I), ledit procédé comprenant les étapes suivantes :
- une étape d'enrichissement d'une huile concentrée en composés de formule (I), à raison d'environ 50% à environ 95%,
   - d'une part, par élimination de tout ou partie des composés apolaires, tels que les triglycérides, les stérols, les cires, les acides gras et autres composés insaponifiables, notamment par partage liquide/liquide de ladite huile concentrée, à l'aide d'un système biphasique de solvants ou par simple déphasage (création de deux phases liquides) dans un mélange hydroalcoolique, éventuellement en présence d'un solvant organique tel que le chloroforme ou le méthylterbutyléther, afin d'obtenir une phase liquide enrichie en composé apolaires susmentionnés et une fraction lipidique liquide enrichie en composés de formule (I), notamment exempte de triglycérides,
   - et, éventuellement, d'autre part, par élimination des composés polaires tels que les sucres résiduels, notamment par partage liquide/liquide de ladite fraction lipidique enrichie en composés de formule (I), telle qu'obtenue précédemment, à l'aide d'un système biphasique de solvants ou par simple déphasage dans un mélange hydroalcoolique, éventuellement en présence d'un solvant organique tel que le chloroforme ou le méthylterbutyléther, ce qui permet d'obtenir une fraction lipidique enrichie en composés de formule (I), exempte de sucres,
   l'ordre des étapes d'élimination des composés apolaires et des composés polaires pouvant être inversé,
- et, éventuellement, une étape de fractionnement de ladite fraction lipidique enrichie en composés de formule (I) ou de ladite fraction lipidique enrichie en composés de formule (I), exempte de sucres, notamment par chromatographie sur gel de silice, par chromatographie liquide sur support solide ou par chromatographie de partage centrifuge, éventuellement par fluides supercritiques, afin d'isoler des sous-fractions contenant au moins un composé de formule (I).

L'expression "huile concentrée" désigne une huile préparée selon un procédé conduisant à une composition avec une teneur en composés de formule (I) variant de 20 à 50%.

L'expression "fraction lipidique liquide enrichie en composés de formule (I)" désigne une fraction comprenant environ 50 à environ 95% de composés de formule (I).

L'expression "composés apolaires" désigne des composés essentiellement hydrocarbonés uniquement solubles dans des solvants de faible constante diélectrique et moment dipolaire tels que les triglycérides, les stérols libres et les alcools gras.

L'expression "composés polaires" désigne ici des composés solubles dans des phases aqueuses tels que les sucres.

L'expression "système biphasique de solvants" désigne un mélange d'au minimum deux solvants dans des proportions conduisant à la formation de deux phases liquides. La formation de deux phases liquides implique l'existence d'un domaine biphasique dans le diagramme de phases dudit système de solvants. A titre d'exemple, le mélange d'un volume de méthanol et d'un volume d'heptane, en présence ou non d'eau conduit à la formation de deux phases liquides.

L'expression "simple déphasage dans un mélange hydroalcoolique" désigne le procédé selon lequel l'addition d'un volume adéquat d'alcool (de préférence d'éthanol) et d'eau à l'huile enrichie permet la formation de deux phases liquides.

L'expression "fluides supercritiques" désigne l'état physique d'un corps dans des conditions de pression et de température (au-delà du point critique) telles que ce corps présente un masse volumique proche de celle d'un liquide et une viscosité proche de celle d'un gaz.

La présente invention concerne également un procédé de préparation d'une composition cosmétique telle que définie ci-dessus et contenant des fractions lipidiques enrichies en composés de formule (I) ou des sous-fractions contenant au moins un composé de formule (I), ledit procédé comprenant les étapes suivantes :
- une étape d'extraction solide/liquide, notamment avec au moins un solvant d'extraction de type polaire ou d'un fluide supercritique, à partir de matière première solide, éventuellement broyée, provenant de plantes, d'algues, de procaryotes ou d'eucaryotes, ladite matière première comprenant au moins un composé de formule (I), afin d'obtenir un mélange hétérogène comprenant, après établissement des équilibres thermodynamiques, une phase solide épuisée des composés de formule (I) et une phase lipidique contenant les composés de formule (I) en solution dans le solvant d'extraction, contenant éventuellement de l'eau,
- une étape de séparation solide/liquide dudit mélange hétérogène tel qu'obtenu à l'étape précédente, afin d'obtenir la phase lipidique seule, en solution dans le solvant d'extraction, contenant éventuellement de l'eau,
- une étape de concentration de ladite phase lipidique, en solution dans le solvant d'extraction, contenant éventuellement de l'eau, afin d'éliminer le ou les solvants d'extraction et/ou l'eau résiduelle, permettant d'obtenir une huile concentrée en composés de formule (I), à raison d'environ 15% à environ 50%,
- une étape d'enrichissement de ladite huile concentrée en composés de formule (I), à raison d'environ 50% à environ 95%,
   - d'une part, par élimination de tout ou partie des composés apolaires, tels que les triglycérides, les stérols, les cires, les acides gras et autres composés insaponifiables, notamment par partage liquide/liquide de ladite huile concentrée, à l'aide d'un système biphasique de solvants ou par simple déphasage dans un mélange hydroalcoolique, éventuellement en présence d'un solvant organique tel que le chloroforme ou le méthylterbutyléther, afin d'obtenir une phase liquide enrichie en composé apolaires susmentionnés et une fraction lipidique liquide enrichie en composés de formule (I), notamment exempte de triglycérides,
   - et, éventuellement, d'autre part, par élimination des composés polaires tels que les sucres résiduels, notamment par partage liquide/liquide de ladite fraction lipidique enrichie en composés de formule (I), telle qu'obtenue précédemment, à l'aide d'un système biphasique de solvants ou par simple déphasage dans un mélange hydroalcoolique, éventuellement en présence d'un solvant organique tel que le chloroforme ou le méthylterbutyléther, ce qui permet d'obtenir une fraction lipidique enrichie en composés de formule (I), exempte de sucres,
- et, éventuellement, une étape de fractionnement de ladite fraction lipidique enrichie en composés de formule (I) ou de ladite fraction lipidique enrichie en composés de formule (I), exempte de sucres, notamment par chromatographie sur gel de silice, par chromatographie liquide ou par chromatographie de partage centrifuge, afin d'isoler des sous-fractions contenant au moins un composé de formule (I).

L'expression "solvant d'extraction de type polaire" désigne un solvant présentant une constante diélectrique telle que 4 < ε < 50 et/ou un moment dipolaire tel que 1 < µ_{(Debye)} < 4). A titre d'exemple, on peut citer les alcools tels que le méthanol, l'éthanol, le propanol, l'isopropanol ou le 1-butanol ; les cétones telles que l'acétone, la méthyl-éthylcétone ou la méthyl-isobutylcétone ; les esters tels que l'acétate d'éthyle ou l'acétate de butyle ; les éthers tels que l'éther diéthylique ou l'éther de méthyle et de tertiobutyle ; les solvants chlorés tels que le chloroforme ou le dichlorométhane ; ou un mélange de ces différents solvants. La présence d'eau est possible, provenant soit de la matière première, soit des solvants d'extraction (éthanol non absolu par exemple).

La présente invention concerne également un procédé de préparation tel que défini ci-dessus, caractérisé en ce que la matière première provient :
- de plantes appartenant à la famille Poaceae, notamment choisies parmi : *Triticum sp., Zea mays, Oryza sativa, Hordeum vulgare, Secale cereale, Panicum sp., Sorghum sp., Fagopyrum sp., Pennisetum sp., Triticum x Secale* et *Avena sp.,* à la famille Brassicaceae telle que *Brassica sp.,* à la famille Cucurbitaceae telle que *Cucurbita maxima,* à la famille Palmeae telle que *Cocos nucifera,* à la famille Solanaceae telle que *Solanum tuberosum,* à la famille Olaceae telle que *Olea europa,* à la famille Chenopodiaceae, notamment choisies parmi : *Beta vulgaris* et *Spinacia oleacera,* à la famille Fabaceae, notamment choisies parmi : *Glycine max* et *Arachis sp.,* à la famille Asteraceae telle que *Helianthus sp.,* à la famille Lauraceae telle que *Perseae americana,* et à la famille Papilionaceae, notamment choisies parmi : *Lupinus sp.* et *Pisum sp.,*
- de procaryotes tels que *Bifidobacterium,*
- d'eucaryotes choisis parmi : *Chlamydomonas sp., Batrachospermum sp.* et *Fucus vesiculosus.*

Un procédé de préparation avantageux de l'invention est caractérisé en ce que l'élimination de tout ou partie des composés apolaires comprend les étapes suivantes :
- une étape d'extraction liquide/liquide de l'huile concentrée telle que définie ci-dessus, avec un système biphasique à base de méthanol, d'heptane et d'eau (entre 0,1 et 10%), ce qui permet l'obtention d'une fraction dans l'heptane, nommée H1a, et d'une fraction méthanolique, nommée M1a, enrichie en composés de formule (I),
- une étape d'extraction liquide/liquide de ladite fraction M1a avec de l'heptane, ce qui permet l'obtention d'une fraction dans l'heptane, nommée H1b, et d'une fraction méthanolique, nommée M1b, qui correspond à une fraction lipidique enrichie en composés de formule (I), et
- éventuellement, une étape d'évaporation et une étape de dissolution dans de l'heptane desdites fractions H1a et H1b, et une étape d'extraction liquide/liquide desdites fractions dans l'heptane avec du méthanol additionné d'eau (entre 0,1 et 10%), ce qui permet l'obtention d'une fraction dans l'heptane, nommée H2, et d'une fraction méthanolique, nommée M2, enrichie en composés de formule (I).

Les fractions H1a et H1b contiennent majoritairement (entre 50% et 95%) des composés apolaires tels que des triglycérides, des stérols et des acides gras, mais il persiste également des composés de formule (I).

La fraction H2 contient essentiellement des composés apolaires (entre 75% et 95%) tels que des triglycérides, des stérols et des acides gras. Elle peut contenir des composés de formule (I-4) où R₆ désigne un groupement acyle.

Les fractions M1a et M1b sont des fractions d'intérêt et contiennent essentiellement (entre 40% et 70%) des composés de formule (I), ainsi que de l'acide tianshique, des cires et des sucres.

La fraction M2 est également une fraction riche en composés de formule (I) (entre 40% et 70%), contenant des composés apolaires résiduels mais exempte de sucres.

Un procédé préféré de l'invention est caractérisé en ce que la fraction M1b telle que définie ci-dessus est soumise à une étape d'élimination des composés polaires tels que les sucres résiduels, notamment par partage liquide/liquide dans le système n-butanol/eau, de ladite fraction M1b, ce qui permet d'obtenir une fraction lipidique enrichie en composés de formule (I), exempte de sucres.

Un procédé préféré de l'invention est un procédé tel que défini ci-dessus, comprenant une étape de fractionnement de la fraction H2 telle que définie ci-dessus, par chromatographie sur gel de silice, afin d'isoler une sous-fraction contenant au moins un composé de formule (I), par exemple un composé de la famille des MAMGDG, dans laquelle R₁ et R₂ représentent un groupe acyle et R₃ représente un groupe de formule (C) telle que définie ci-dessus.

Selon un mode de réalisation avantageux, le procédé de l'invention comprend une étape de fractionnement de la fraction lipidique enrichie en composés de formule (I), exempte de sucres, par chromatographie de partage centrifuge, en utilisant les systèmes biphasiques suivants : heptane/acétate d'éthyle/acétone/méthanol/eau, notamment dans les proportions volumiques 2:2:2:1:1 et heptane/acétonitrile/n-butanol, notamment dans les proportions volumiques 6:3,8:1,2.

Un mode de réalisation avantageux de l'invention est caractérisé en ce que l'huile de gluten de blé est dissoute dans un mélange de chloroforme de méthanol et d'eau (6:7:5 v/v) dans les proportions suivantes : 3,6 litres de système de solvants/kg d'huile. Il en résulte la formation de deux phases, une phase inférieure et une phase supérieure contenant essentiellement des sucres libres.

Un mode de réalisation avantageux de l'invention est caractérisé en ce que la phase inférieure riche en chloroforme telle que définie ci-dessus est additionnée d'un mélange de méthanol et d'eau (2:1, v/v ; 1,2 litre /kg d'huile de départ), la phase supérieure, riche en composés de formule (I) étant gardée. La phase inférieure contenant encore des composés de formule (I) est extraite à nouveau deux fois par le mélange méthanol/eau. La phase inférieure est soumise deux fois au même procédé que décrit ci-dessus en utilisant cette fois un mélange méthanol/eau (3:1, v/v ; 1,6 litre /kg d'huile de départ). Les phase supérieures, contenant essentiellement des composés de formule (I), y compris les composés de formule (I-4) où R₆ représente un groupement acyle, sont rassemblées. Elles représentent 60% de l'huile de gluten de blé de départ.

La présente invention concerne également un procédé tel que défini ci-dessus, dans lequel l'huile concentrée est obtenue selon un procédé comprenant les étapes suivantes :
- une étape d'extraction solide/liquide, notamment avec de l'éthanol, à partir de gluten de blé, afin d'obtenir un mélange hétérogène comprenant une phase solide et une phase lipidique en solution dans l'éthanol, contenant éventuellement de l'eau,
- une étape de séparation solide/liquide dudit mélange hétérogène tel qu'obtenu à l'étape précédente, permettant d'éliminer le gluten délipidé, afin d'obtenir la phase lipidique seule, en solution dans l'éthanol, contenant éventuellement de l'eau, et
- au moins une étape de concentration de ladite phase lipidique, en solution dans l'éthanol, contenant éventuellement de l'eau, afin d'éliminer l'éthanol et/ou l'eau résiduelle, permettant d'obtenir une huile concentrée en composés de formule (I), telle que définie ci-dessus.

Les étapes de fractionnement sont justifiées car elles permettent non seulement d'enrichir l'extrait en principes actifs mais également d'augmenter l'activité de la fraction M1(-sucres) vis-à-vis de la MMP-1. Dans une moindre mesure, elles augmentent l'interaction de la fraction M1(-sucres) avec la plasmine, laissant présager de la présence de composés pouvant inhiber cette enzyme dans l'huile brute (tableaux ci-dessous).

**Tableau : Comparaison entre les facteurs d'enrichissement liés au fractionnement et les augmentations d'activité au cours du même fractionnement sur l'activité amidolytique de la plasmine**

| | **% en composés d'intérêts*** | **Facteur d'enrichissement** | **Action sur l'activité de la plasmme (Vᵢ/V₀) à isoconcentration** | **Augmentation d'activité** |
|---|---|---|---|---|
| huile de gluten de blé (HGB) | 35 | 1,00 | 1,17 | 1,00 |
| M1 | 60 | 1,71 | 1,45 | 1,24 |
| M1(-sucres) | 75 | 1,25 | 3,58 | 2,47 |
| ED 23-11 (MAMGDG) | 100 | 1,33 | 8,44 | 2,36 |

| | | | | |
|---|---|---|---|---|
| *: estimé en fonction des résultats des fractionnements par CPC et des rendements des fractionnements liquide/liquide | | | | |

**Tableau : Comparaison entre les facteurs d'enrichissement liés au fractionnement et les augmentations d'activité au cours du même fractionnement sur l'inhibition de l'activité de la collagénase de type I**

| | **% en composés d'intérêts*** | **Facteur d'enrichissement** | **Action sur l'activité de la MMP-1 (Vᵢ/V₀) à isoconcentration** | **Augmentation d'activité** |
|---|---|---|---|---|
| huile de gluten de blé (HGB) | 35 | 1,00 | 10 | 1,00 |
| M1 | 60 | 1,71 | 27,1 | 2,71 |
| M 1(-sucres) | 75 | 1,25 | 61,8 | 2,28 |
| ED 23-11 (MAMGDG) | 100 | 1,33 | 168 | 2,72 |

### DESCRIPTION DES FIGURES

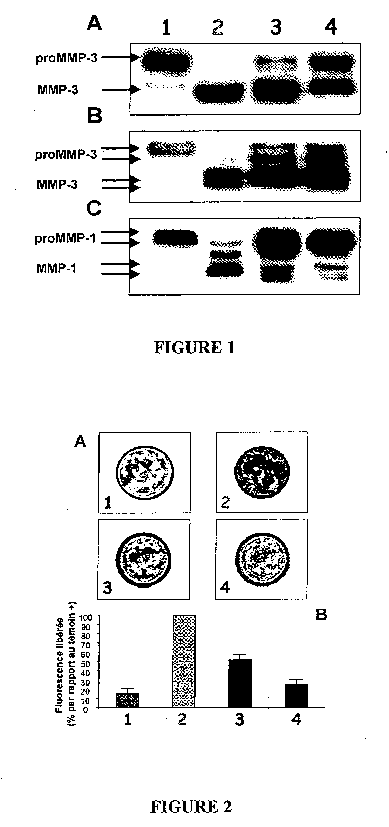

La Figure 1 représente la modulation de l'activation des proMMP-3 (Figures 1A et 1B) et proMMP-1 (Figure 1C) *in vitro* comme en culture par des extraits glycolipidiques du gluten de blé.
   Figure 1A : 100 nM de proMMP-3 (Figure 1A-1) sont incubés avec 65 nM de plasmine en absence (Figure 1A-2) ou en présence de glycolipides (Figure 1A-3 et 4), à la concentration de 50µg/mL pour M1-Sucres (Figure 1A-3) et 25 µg/mL pour ED 23-11 (Figure 1A-4).
   Figure 1B : des fibroblastes dermiques sont ensemencés en plaque 24 puits à raison de 105 cellules par puits jusque confluence. Après une mise au repos de 18 heures, les cellules sont incubées en présence (Figure 1B-2, 3 et 4) ou non (Figure 1B-1) de 2,5 µg/mL de plasmine ainsi que des glycolipides M1(-Sucres) (Figure 1B-3) et ED 23-11 (Figure 1B-4) aux mêmes concentrations que précédemment.
   Figure 1C : des fibroblastes dermiques sont ensemencés en plaque 24 puits à raison de 105 cellules par puits jusque confluence. Après une mise au repos de 18 heures, les cellules sont incubées en présence (Figure 1C-2, 3 et 4) ou non (Figure 1C-1) de 2,5 µg/mL de plasmine ainsi que des glycolipides M1(-Sucres) (Figure 1C-3) et ED 23-11 (Figure 1C-4) aux mêmes concentrations que précédemment.
   Dans l'ensemble des figures 1A, 1B et 1C, la réaction est arrêtée aux temps indiqués par addition de 0,3 mg/ml d'aprotinine. Les échantillons sont ensuite soumis à une électrophorèse en gel de polyacrylamide à 10% (p/v) et à une immuno-révélation à l'aide d'un anticorps anti-MMP-3 (Figure 1A et 1B) et anti-MMP-1 (Figure 1C).
La Figure 2 représente les effets de la fraction M1(-Sucres) et du composé ED 23-11 (MAMGDG) sur la dégradation d'un tapis de collagène dans un modèle de photo-vieillissement cutané.
   Les Figures 2A-1, 2A-2, 2A-3 et 2A-4 représentent l'observation de la dégradation du tapis de collagène sous lampe UV.
   Les Figures 2B-1, 2B-2, 2B-3 et 2B-4 représentent la quantification de cette dégradation (pourcentage de fluorescence libérée par rapport au témoin).
   Les Figures 2A-1 et 2B-1 correspondent au test dans lequel les cellules ne sont pas photo-irradiées par des UV-A ; les Figures 2A-2 et 2B-2 correspondent au test dans lequel les cellules sont photo-irradiées par des UV-A et dans lequel le milieu est supplémenté par 15 µg/mL de plasminogène ; les Figures 2A-3 et 2B-3 correspondent au test dans lequel les cellules sont photo-irradiées par des UV-A et dans lequel le milieu est supplémenté par 15 µg/mL de plasminogène et par M1(-Sucres) à 50 µg/mL ; et les Figures 2A-4 et 2B-4 correspondent au test dans lequel les cellules sont photo-irradiées par des UV-A et dans lequel le milieu est supplémenté par 15µg/mL de plasminogène et par ED 23-11 à 25 µg/mL.
La Figure 3 représente les effets de la fraction M1(-sucres) et de ED 23-11 sur la synthèse des collagènes totaux (CPM pour 2.10⁵ cellules). La Figure 3-1 correspond à la mesure témoin ; la Figure 3-2 correspond à l'effet de la fraction M1(-sucres) et la Figure 3-3 correspond à l'effet du composé ED 23-11. Les colonnes pleines correspondent au dosage du collagène dans le milieu de culture et les colonnes hachurées à celui réalisé au niveau cellulaire.
La Figure 4 représente le diagramme de préparation de l'huile de gluten de blé.
La Figure 5 représente le diagramme correspondant à l'enrichissement de l'huile de gluten de blé en lipides polaires.
La Figure 6 représente le spectre RMN de composés de la famille des DGMG.
La Figure 7 représente le spectre RMN de composés de la famille des MAG.
La Figure 8 représente le spectre RMN de composés de la famille des MGDG.
La Figure 9 représente le spectre RMN de composés de la famille des DGDG.

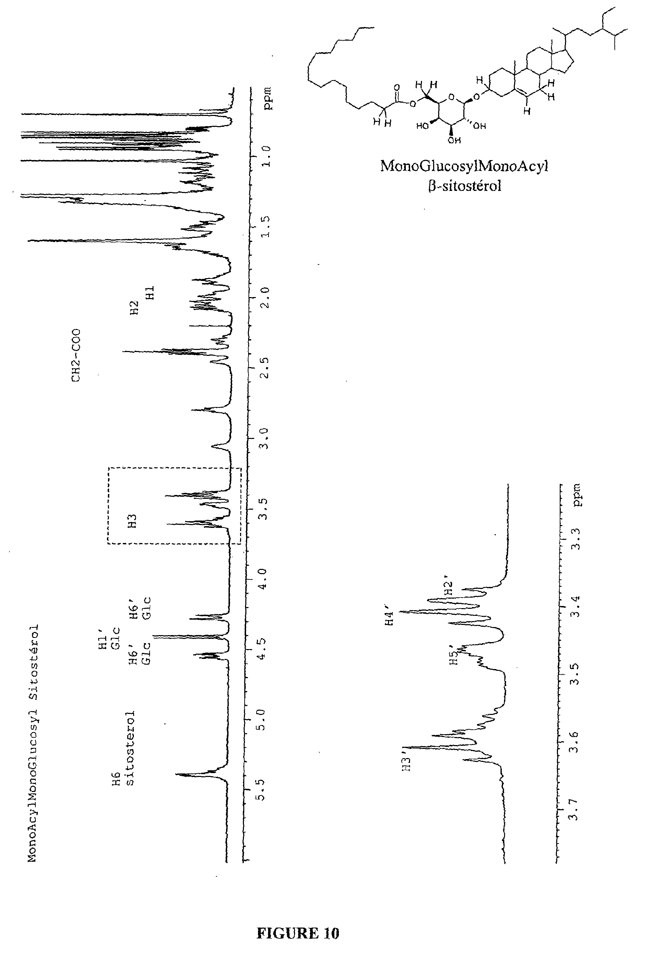

La Figure 10 représente le spectre RMN du monoglucosylmonoacyl β-sitostérol.

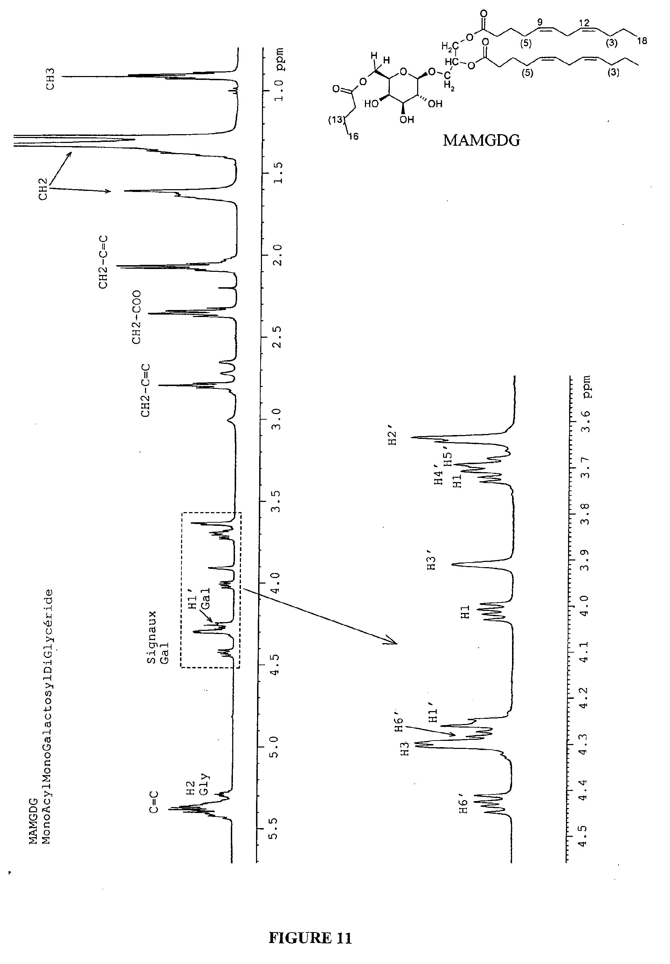

La Figure 11 représente le spectre RMN de composés de la famille des MAMGDG.

### PARTIE EXPÉRIMENTALE - CHIMIE

### 1 - PROTOCOLE GÉNÉRAL D'EXTRACTION

Ce protocole est décomposé en opérations unitaires.

### 1. Fragmentation en solides finement divisés

Afin de faciliter l'étape d'extraction liquide/liquide, il peut être nécessaire, dans le cas par exemple d'une extraction de plante ou d'algue, de fragmenter la matière première en solides finement divisés. Les technologies utilisées sont de type : moulin, broyeur, dilacérateur...

### 2. Extraction solide/liquide

### 2.1. La technologie

Cette étape consiste à extraire par un solvant approprié les composés d'intérêt, à savoir les composés de formule (I) telle que définie ci-dessus, de la matière première lorsque celle-ci est solide (à l'exclusion des bruts de synthèse). Cette extraction peut mettre en oeuvre des technologies impliquant un processus d'extraction de type statique (cuve de macération, agitée ou non) ou dynamique (percolateurs, extracteurs continus à contre-courant type Vatron-Mau par exemple). On peut également envisager l'assistance des micro-ondes afin d'améliorer la cinétique d'extraction et/ou le rendement.

Cette étape peut également consister en une extraction par un fluide supercritique (CO₂ avec ou sans modificateur de polarité tel que le méthanol).

### 2.2. Les solvants

Le liquide d'extraction devra comprendre au moins un solvant présentant :
- une constante diélectrique telle que 4 < ε < 50
- et/ou un moment dipolaire tel que 1 < µ_{(Debye)} < 4)
pour permettre l'extraction des composés d'intérêt répondant à la formule générale (I).

A titre d'exemple, on peut citer les alcools tels que le méthanol, l'éthanol, le propanol, l'isopropanol ou le 1-butanol ; les cétones telles que l'acétone, la méthyl-éthylcétone ou la méthyl-isobutylcétone ; les esters tels que l'acétate d'éthyle ou l'acétate de butyle ; les éthers tels que l'éther diéthylique ou l'éther de méthyle et de tertiobutyle ; les solvants chlorés tels que le chloroforme ou le dichlorométhane ; ou un mélange de ces différents solvants.

La présence d'eau est possible, provenant soit de la matière première, soit des solvants d'extraction (éthanol non absolu par exemple).

Au cours de cette étape d'extraction solide/liquide, l'effet bénéfique de la température peut être mis a profit, sachant que la température de travail doit être fixée en fonction du liquide d'extraction. A titre d'exemple, l'extraction peut se faire à l'éthanol 99,9%, à 40°C, pendant 3 heures dans une cuve à macération.

### 3. Séparation solide/liquide

### 3.1. La technologie

Le résultat de l'étape précédente est un mélange hétérogène constitué du solide épuisé des composés d'intérêt et de la solution extractive contenant les composés d'intérêt. Les phases solide et liquide doivent être séparées en appliquant les technologies suivantes :
- le criblage (grille courbe, tamis,...)
- la séparation gravitaire (décantation statique ou hydrocyclonage)
- la centrifugation (essoreuse, décanteuse)
- la filtration frontale (sous vide ou sous pression, avec ou sans adjuvant de filtration)
- la filtration tangentielle (microfiltration si les particules de solide ne sont pas colmatantes).

Le résultat de cette opération unitaire est l'obtention d'un résidu solide appelé gâteau et d'une solution extractive contenant les composés d'intérêt.

Cette solution extractive pourra être directement évaporée (évaporateurs à circulation forcée, à flot tombant, à grimpage, à couche mince, à surface raclée, à effets simples ou multiples, sous vide ou sous pression, bouilleurs) afin d'éliminer les solvants d'extraction et/ou l'eau résiduelle pour fournir un extrait "sec". Ce dernier peut être utilisé directement ou être engagé dans des étapes de lavage par partage liquide/liquide telles que décrites ci-après.

### 4. Lavage de la solution extractive

En première intention, un changement de température appliqué à la solution extractive peut faciliter la séparation des phases lipidiques et/ou des impuretés par sédimentation. A titre d'exemple, dans le cas d'une extraction à l'éthanol, un refroidissement de la solution extractive permet le déphasage de la phase lipidique riche en composés d'intérêt.

De même, l'adjonction à la solution extractive d'un solvant moins polaire que le solvant d'extraction permet de précipiter sélectivement les sucres résiduels.

Des extractions liquide/liquide successives, utilisant des techniques classiques (type cuve à agitation puis décantation gravitaire, décanteuses liquide/liquide à un ou plusieurs étages, colonnes d'extraction liquide/liquide, Podbielniak (www.techniques-ingenieur.fr) permettent d'enrichir l'extrait en composés d'intérêt en éliminant :
- les composés apolaires, tels que les triglycérides, les stérols, les cires, les acides gras et autres insaponifiables : ce fractionnement se fait, par exemple, en utilisant le système biphasique heptane/méthanol auquel on ajoute un faible pourcentage d'eau (environ 5%) ; les composés d'intérêt se partagent majoritairement en faveur de la phase méthanolique alors que les composés apolaires se partagent en faveur de la phase heptane ;
- les composés polaires, tels que les sucres résiduels par partage liquide/liquide, en utilisant un système biphasique ayant une phase riche en eau : par exemple, le système 1-butanol/eau permet de solubiliser les sucres dans la phase aqueuse alors que les composés d'intérêt se partagent en faveur de la phase organique.

### II - MISE EN OEUVRE DU PROCÉDÉ AVEC L'HUILE DE GLUTEN DE BLÉ

Le blé tendre et le blé dur sont les 2 céréales significativement plus riches en gluten que les autres graminées ou céréales de grandes cultures. Le gluten de blé est un réseau protéique aux propriétés viscoélastiques remarquables, aux applications agro-alimentaires très importantes. Les propriétés sont intrinsèquement liées à la présence très concentrée de lipides (74% des lipides de la farine de blé). Les interactions lipides-protéines sont très spécifiques du gluten de blé, et ne sont pas communément répandues chez d'autres espèces. Ces lipides sont majoritairement des glycolipides et des lipides neutres, les phospholipides étant majoritairement répartis dans la partie amylacée de la farine.

Le gluten représente 10% de la masse de la farine totale, mais entraîne avec lui 74% des lipides. Il en résulte que l'extraction des galactosylglycérides à partir du gluten a un sens économique, puisqu'il faut traiter 10 fois moins de volume (et par voie de conséquence, utiliser moins de solvants, d'une manière optimale) pour extraire une fraction plus pure et plus concentrée dans les molécules d'intérêts et générer 10 fois moins de coproduits pollués par les solvants d'extraction.

Il est donc avantageux de démarrer l'extraction à partir du gluten riche en lipide.

### 1- Préparation de l'huile de gluten de blé

Cette étape concerne le processus d'extraction solide-liquide tel que décrit ci-dessus, suivi par une étape d'essorage puis deux étapes de concentration. L'huile est obtenue avec un rendement de 4,1 % par rapport à la matière sèche de départ.

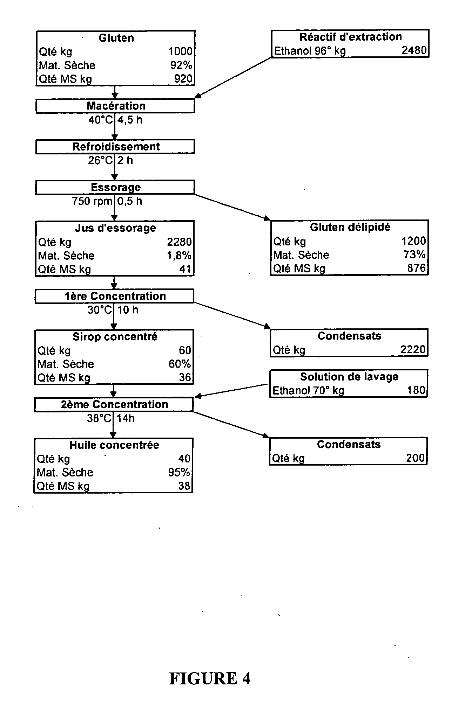

Ce processus peut être représenté par le diagramme de la Figure 4.

Cette extraction a été reproduite sur différents lots de gluten.

### Mode opératoire :

Une tonne de gluten de blé, contenant 8% d'eau résiduelle, est mise à macérer dans 3 100 litres d'éthanol à 99%, à 40°C, pendant 4 heures et 30 minutes. Le mélange est ensuite refroidi à 26°C, puis maintenu à cette température pendant 2 heures. Le mélange est alors introduit dans une essoreuse (les essoreuses utilisées peuvent être de tout type et de toutes tailles, celle utilisée avantageusement fonctionnant entre 1 500 et 4 500 g, pour un diamètre de 800 à 1 000 mm, et étant pourvue d'une toile filtrante en matériaux naturels ou synthétiques d'une porosité comprise entre 10 et 250 µm), puis soumis à une rotation de 750 tpm pendant 30 minutes. Le gluten délipidé (1 200 kg, contenant 27% de solvant résiduel) et la solution extractive (2 280 kg, contenant 41 kg de matière sèche) sont ainsi séparés. Une première concentration de la solution extractive est effectuée dans un évaporateur à deux étages, à effets simples, à circulation forcée dans des échangeurs de chaleur à plaques, sous une température de 30°C pendant 10 heures. Un sirop concentré (60 kg, contenant 60% de matière sèche) est alors obtenu. Ce sirop est repris par 207 litres d'éthanol à 70%, puis une deuxième concentration est effectuée à une température de 38°C pendant 14 heures. 40 kg d'huile de gluten de blé, contenant 5% de solvants résiduels, sont alors obtenus.

### 2- Fractionnement liquide-liquide de l'huile de gluten de blé

### a - Enrichissement en lipides polaires

Cette étape a pour but d'enrichir l'huile obtenue précédemment en composés d'intérêt (acylglycérides glycosylés ou non - composés de formule (I) telle que définie ci-dessus).

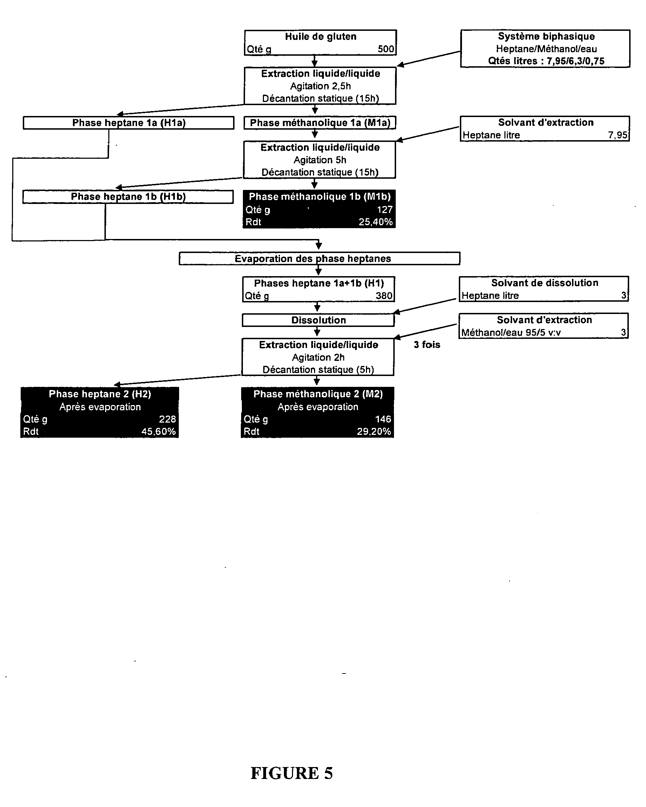

L'extrait obtenu est nommé M1b et il représente 25,4% de l'huile de gluten de blé. Le protocole d'extraction est présenté dans la Figure 5.

### b - « Dé-sucrage » de l'extrait M1b

L'extrait M1b (5,5 g) est soumis à un partage liquide/liquide dans le système n-butanol/eau (1,5 1). Deux extraits sont obtenus après évaporation :
- l'extrait contenant les sucres : 1,86 g
- l'extrait contenant les composés d'intérêt, nommé M(-sucres) : 3,85 g (soit 16% de l'huile brute)

### Description détaillée du mode opératoire :

500 g d'huile de gluten de blé sont dissous dans 15 litres du système biphasique heptane/méthanol/eau 53:42:5 v/v. Le mélange est agité dans un conge, à l'aide d'une pale d'agitation pendant 2 heures et 30 minutes, puis est transféré par une pompe dans une ampoule à décanter. Après 15 heures, la décantation permet une séparation des deux phases. La phase lourde (riche en méthanol) est re-extraite par 7,950 litres d'heptane. Après agitation, décantation et transfert dans une ampoule à décanter, les deux phases sont séparées. Les phases riches en heptane sont rassemblées. Les phases riches en méthanol et riches en heptane sont évaporées sous pression réduite pour fournir les extraits respectivement appelés M1 (126 g) et H1 (380 g). H1 est repris dans 3 litres d'heptane puis extrait (agitation, décantation, séparation) par 3 fois 3 litres du mélange méthanol/eau 95:5 v/v. Les deux phases résultantes, riches en méthanol ou en heptane sont respectivement nommées H2 (228 g) et M2 (146 g). Les fractions M1 et M2 sont riches en composés d'intérêt : les glycosylglycérides, répondant à la formule (I) susmentionnée.

5,5 g de la fraction M1 sont dissous dans 1,5 litres du système biphasique butanol/eau afin de séparer les sucres et les glycosylglycérides. Après agitation, décantation et séparation, la phase butanolique est mise à sec pour fournir 3,85 g d'extrait enrichi en composés d'intérêt nommée M1(-sucres).

### 3- Suivi analytique des glycolipides

### Chromatographie sur couche mince (CCM) à polarité de phases inversées

Cette technique permet un suivi analytique qualitatif rapide des composés d'intérêt : les glycosyl-glycérides (formule (I) telle que définie ci-dessus). Il s'agit de chromatographie planaire utilisant un support de type silice greffée C 18 et une phase mobile constituée d'un mélange méthanol/acétate d'éthyle 96 :4 (v/v).

### CLHP

Cette technique permet un suivi analytique qualitatif et quantitatif des composés d'intérêt. La phase stationnaire utilisée est de type polymérique polystyrène-divinyl-benzène (PLRP-S, 250 x 4,5, PolymèreLab). La phase mobile est constituée d'acétonitrile et d'eau, en mode gradient. La détection est assurée par un détecteur évaporatif à diffusion de lumière.

### 4- Isolement de glycolipides par chromatographie

Le but de ces étapes est de caractériser au mieux les composés contenus dans les différents extraits et de tenter de dégager des relations structure/activité.

Les techniques de chromatographie utilisées sont de plusieurs types :
- la chromatographie d'adsorption sur gel de silice pour l'extrait H2 ;
- la chromatographie de partage centrifuge (CPC) pour l'extrait M(-sucres) ;
- la CLHP semi préparative à polarité de phases inversée (phase polymérique) couplée à un détecteur à diffusion de lumière pour les fractions obtenues à partir de M(-sucres).

### a - Fractionnement de H2 :

Le fractionnement de H2 par chromatographie sur gel de silice a permis d'isoler le β-sitostérol glycosylé et le 2,3-di-O-linoléyl-1-O-[6'-O-palmitoyl-β-D-galactopyranosyl]-D-glycérine (MAMGDG, ED23-11).

### Mode opératoire détaillée concernant H2 :

### Fractionnement d'un mono-acyl-mono-galactosyl-diglycéride (MAMGDG) par colonne sur gel de silice de H2

10 g de H2 sont fractionnés par flash chromatographie (technique de chromatographie sous basse pression) sur gel de silice, en appliquant un gradient d'élution par paliers, la phase mobile étant constituée d'un mélange de cycohexane/chloroforme (50/50), puis de chloroforme, puis d'un mélange chloroforme/acétone (90:10, puis 85:15, puis 80:20, puis 70:30), et enfin du mélange acétone/méthanol (50:50). La fraction éluée avec la phase mobile chloroforme/acétone 90:10 (454 mg) est à nouveau soumise à une étape de flash chromatographie sur gel de silice en appliquant un gradient de phase mobile constitué des mêmes solvants que précédemment. Un composé (340 mg, soit environ 1,5% de l'huile de gluten de blé) est élué pur avec une phase mobile constitué du mélange chloroforme/acétone 98:2. L'analyse par résonance magnétique nucléaire et par spectrométrie de masse permet d'élucider sa structure : il s'agit du 2,3-di-O-linoléyl-1-O-[6'-O-palmitoyl-β-D-galactopyranosyl]-D-glycérine, de la famille des monoacyl-monogalactosyl-diglycérides (MAMGDG, référencé ED23-11 pour les tests biologiques).

### b - Fractionnement de M(-sucres) :

Le fractionnement de M(-sucres) par CPC et par CLHP a permis d'isoler différentes familles de dérivés du glycérol. Il faut noter que les composés sont isolés sous forme de mélanges d'homologues.

Les fractions enrichies testées ont été obtenues en utilisant la CPC avec les systèmes biphasiques suivants : heptane/acétate d'éthyle/acétone/méthanol/eau 2:2:2:1:1 v/v et heptane/acétonitrile/*n*-butanol 6:3.8:1.2 v/v.

Les structures des composés suivants ont été déterminées à ce jour :
- les mono-acyl-glycérides (MAG), avec essentiellement de l'acide linoléique et en quantité minoritaire de l'acide palmitoléique : il s'agit des 1-O-(palmitoyl
ou linoléyl)-D-glycérine ;
- les mono-galactosyl-mono-glycérides (MGMG), avec acides linoléique et palmitoléique ;
- un di-galactosyl-mono-glycéride (DGMG), avec acide linoléique : 3-O-linoléyl-1-O-[6'-O-D-galactopyranosyl-β-D-galactopyranosyl]-D-glycérine ;
- les mono-galactosyl-di-glycérides (MGDG), avec un composé présentant des chaînes grasses saturées, les autres présentant majoritairement des chaînes grasses de type acide linoléique ; un composé isolé est le 2,3-di-O-linoléyl-1-O-β-D-galactopyranosyl-D-glycérine ;
- les di-galactosyl-di-glycérides (DGDG) : 2,3-di-O-(acides gras saturés ou linoléique ou palmitoléique)-1-O-[6'-O-D-galactopyranosyl-β-D-galactopyranosyl]-D-glycérine.

Ont été également isolés :
- des cires
- de l'acide tianschique

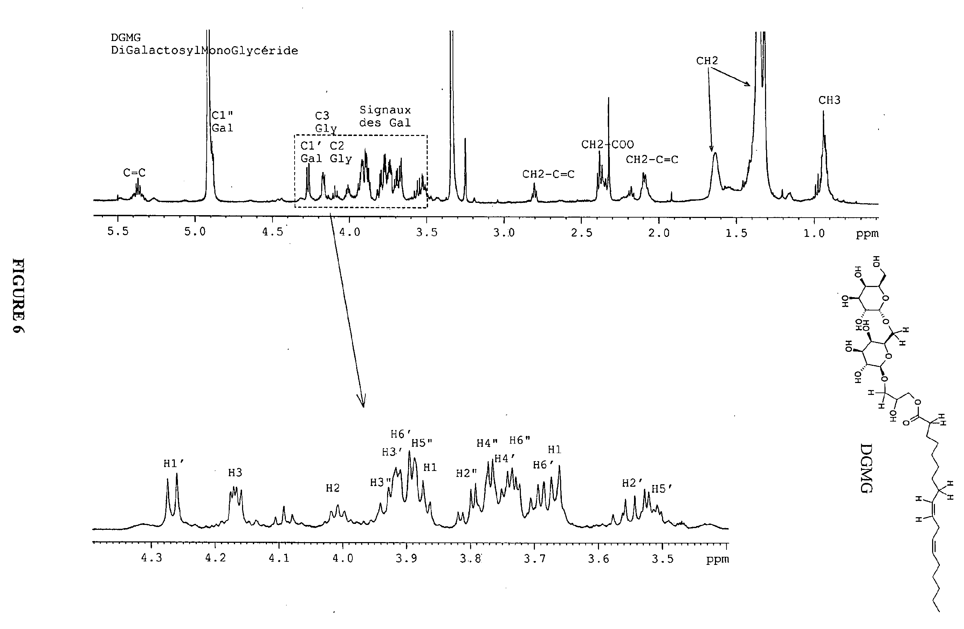

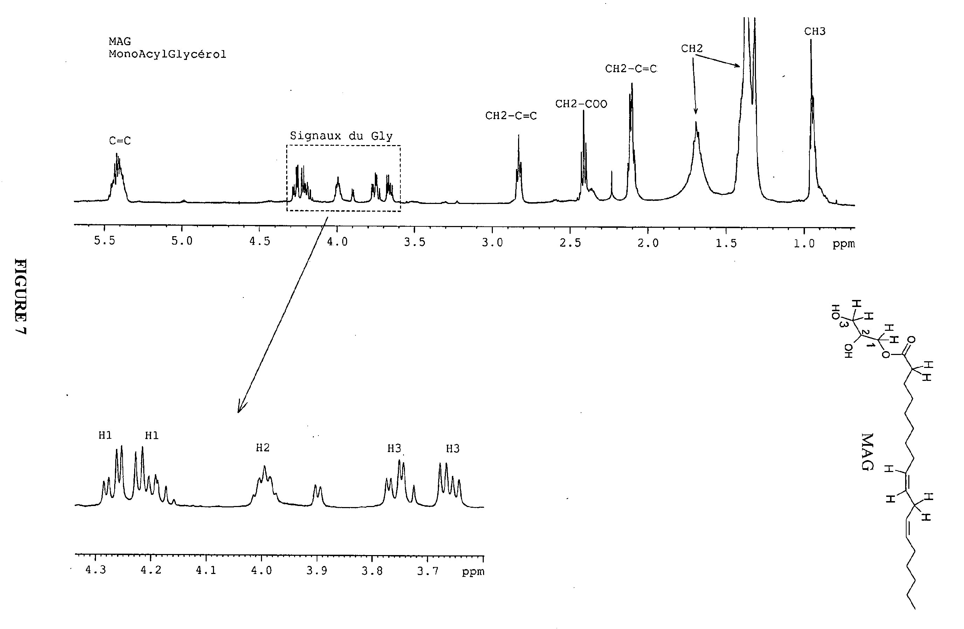

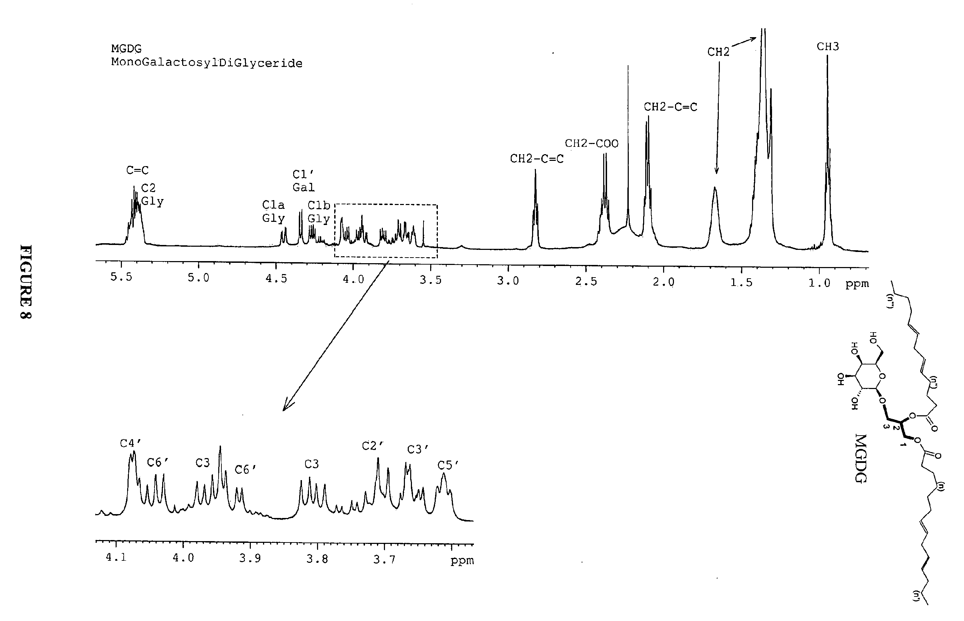

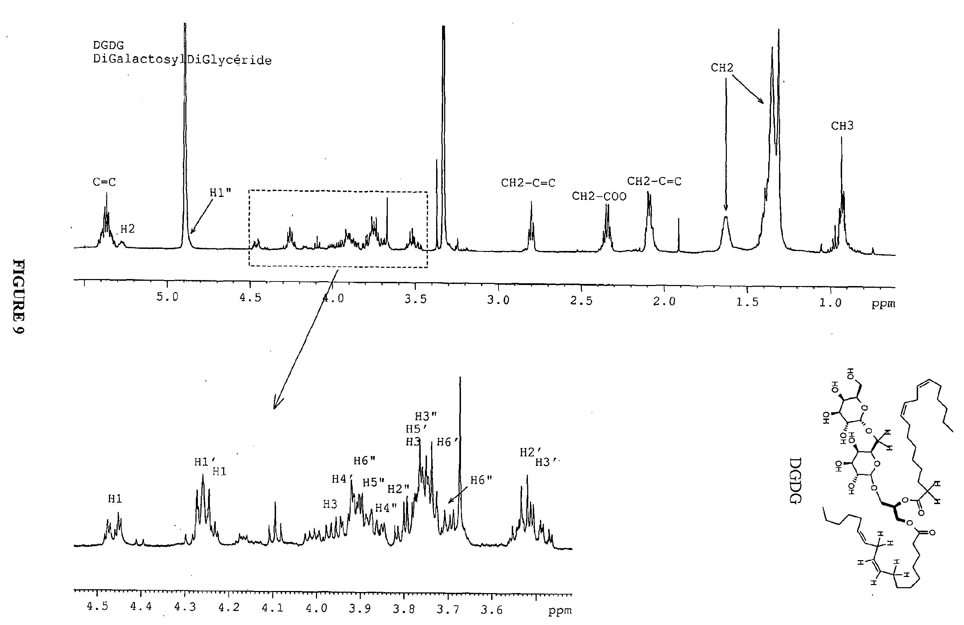

Les déterminations de structures ont été réalisées par spectroscopie de RMN (Figures 4 à 9), mono- et bi-dimensionnelle, mono- et hétéro-nucléaire, et par spectrométrie de masse (mode electrospay).

### Mode opératoire détaillé :

### Fractionnement par chromatographie de partage centrifuge de M1(-sucres)

La chromatographie de partage centrifuge (CPC) est une technique de chromatographie liquide-liquide sans support solide (Foucault, A.P. (1994) Ed. Centrifugal Partition Chromatography - Theory and Practice, Marcel Dekker, New York ; Berthod, A. (2002) Ed. Countercurrent chromatography - The support-free liquid phase, Elsevier Science B.V.), qui requiert l'utilisation d'un système biphasique élaboré à partir d'au moins deux solvants et/ou solutions.

### 1) Système heptane / acétate d'éthyle / acétone / méthanol / eau (expérience 1)

### a- Système biphasique de solvants :

Le système utilisé correspond au mélange heptane/acétate d'éthyle/acétone/ méthanol/eau 2:2:2:1:1 (v/v). Les solvants sont agités dans une ampoule à décanter, puis séparés. La phase organique est choisie comme phase mobile, la phase aqueuse comme phase stationnaire.

### b- Séparation par CPC (expérience 1) :

460 mg d'extrait M1(-sucres) obtenu selon la méthode décrite ci-dessus sont dissous dans 10 ml de phase stationnaire aqueuse et 10 ml de phase mobile organique.

La colonne est remplie de phase stationnaire aqueuse (300 tours/min, mode ascendant, 20 ml/min), puis la rotation est fixée à 1 500 tours/min. Le volume d'injection est alors introduit dans la colonne via une boucle d'injection puis poussé par la phase mobile à 5 ml/min. L'élution en mode ascendant est poursuivie pendant 2 heures, le pourcentage de rétention de la phase stationnaire étant de 64% et la perte de charge de 40 bars. Au bout de 2 heures, les phases mobiles et stationnaires sont inversées, pour cette fois effectuer une élution en mode descendant. Ce type d'inversion est appelée "dual-mode" (Renault JH, Nuzillard JM, Intes O, Maciuk A, Solvent systems in : Countercurrent chromatography : the support free liquid stationary phase. Comprehensive Analytical Chemistry Vol. XXXVIII, Berthod A. Ed., Amsterdam : Elsevier, 2002, pp49-83). Le mode descendant est poursuivi pendant 25 minutes. Cette opération a pour but d'éluer les composés les plus polaires qui sont très retenus par la phase stationnaire initiale, c'est-à-dire la phase aqueuse. L'effluent est fractionné grâce à un collecteur automatique de fractions (SuperFrac Pharmacia). La détection s'effectue en UV (210 nm). L'analyse des fractions par CCM à polarité de phases inversées permet de détecter celles contenant les glycosylglycérides d'intérêt de formule (I). Les tubes présentant le même profil d'élution en CCM sont rassemblées, puis évaporées sous pression réduite.

Le bilan massique est reporté dans le tableau ci-dessous :

| Tubes rassemblés (1min/tube) | Masse en mg |
|---|---|
| 0-14 | 2 |
| 15-20 | 40 |
| 21-22 | 15 |
| **23-25** | 15 |
| 26-27 | 6 |
| **28-31** | 12 |
| **32-39** | 16 |
| 40-51 | 22 |
| 52-53 | 8 |
| 54-57 | 4 |
| **58-67** | 24 |
| 68-69 | 6 |
| 70-73 | 52 |
| 74-75 | 15 |
| **76-80** | 11 |
| 81-86 | 14 |
| **87-93** | 19 |
| 94-102 | 23 |
| 103-106 | 18 |
| 107-112 | 13 |
| 113-115 | 3 |
| 116-120 | 3 |
| 121-124 | 24 |
| 125-127 | 30 |
| 128-132, 133-145 | 62 |

Les fractions ayant été évaluées biologiquement sont en gras dans le tableau.

Après analyse des fractions par RMN, il apparaît que l'extrait injecté contient entre 70 et 80% de glycosylglycérides et de monoacylglycérides.

### c- Deuxième séparation par CPC utilisant le système heptane / acétate d'éthyle / méthanol/eau / acétone 2:2:1:1:2 (expérience 2) :

Le mode opératoire est identique à l'expérience ci-dessus, les paramètres expérimentaux étant reportés dans le tableau suivant :

| Paramètres | Valeurs |
|---|---|
| Masse injectée de M1(-sucres) | 820 mg |
| Mode | Ascendant |
| Rotation | 1800 tpm |
| Débit | 5 ml/mn |
| Volume de phase mobile, Perte de charge | 60, 42 |
| Dual mode | 151 |

Collecteur 1 tube / 35 sec. 171 fractions de 3 ml sont obtenues.

Le bilan massique est reporté dans le tableau ci-dessous :

| Fractions rassemblées (35 sec/tube) | Masse en mg |
|---|---|
| 40-42 | 27 |
| **43-44** | 19 |
| 45-49 | 30 |
| 50-52 | 16 |
| 53-57 | 17 |
| 58-63 | 21 |
| **64-66** | 9 |
| 67-70 | 11 |
| 71-77 | 17 |
| 78-83 | 14 |
| 93-100 | 21 |
| 108-119 | 24 |
| 120-124 | 8 |
| **125-136** | 16 |
| 137-151 | 13 |
| 152 | 21 |
| 153-154 | 52 |
| **155** | 17 |
| 156-157 | 44 |
| 158-160 | 30 |
| 161-164 | 23 |
| 165-171 | 16 |
| 172-175 | 19 |
| 176-179 | 7 |
| 186-200 | 4 |
| 201-fin | 2 |

Les fractions ayant été évaluées biologiquement sont en gras dans le tableau.

### 2) Système Heptane/Acétonitrile/Butanol

### a- Système biphasique de solvants :

Le système utilisé correspond au mélange Heptane/Acétonitrile/Butanol, 60/38/12 (v/v). Les solvants sont agités dans une ampoule à décanter, puis séparés. La phase organique est choisie comme phase mobile, la phase aqueuse comme phase stationnaire.

### b- Séparation par CPC : (expérience 3)

680 mg d'extrait M1(-sucres) obtenu selon la méthode décrite ci-dessus sont dissous dans 10 ml de phase stationnaire aqueuse et 10 ml de phase mobile organique.

La colonne est remplie de phase stationnaire aqueuse (300 tours/min, mode ascendant, 20 ml/min), puis la rotation est fixée à 1 200 tours/min. Le volume d'injection est alors introduit dans la colonne via une boucle d'injection puis poussé par la phase mobile à 5 ml/min. L'élution en mode ascendant est poursuivie pendant 110 minutes, le pourcentage de rétention de la phase stationnaire étant de 54% et la perte de charge de 44 bars. Au bout de 110 minutes, les phases mobiles et stationnaires sont inversées, pour cette fois effectuer une élution en mode descendant ("dual-mode"). L'effluent est fractionné grâce à un collecteur automatique de fractions (SuperFrac Pharmacia). La détection s'effectue en UV (210 nm). L'analyse des fractions par CCM à polarité de phases inversée montre que ce système conduit à une séparation intéressante entre les composés d'intérêt, les glycosyglycérides (et les monoacylglycérides) et les autres métabolites secondaires tels que les triglycérides ou les acides gras. Les fractions comprenant les composés d'intérêt et présentant le même profil en CCM sont rassemblées, évaporées sous pression réduite, puis pesées.

Le bilan massique est reporté dans le tableau ci-dessous :

| Tubes rassemblés (35 sec/tube) | Masse en mg |
|---|---|
| 0-28 | 0,4 |
| 29-32 | 211,3 |
| 33-39 | 7,8 |
| **40-56** | 13,9 |
| 57-68 | 13,1 |
| 69-92 | 11 |
| 93-109 | 7 |
| 110-138 | 11 |
| 139-147 | 6 |
| 148-161 | 6 |
| 162-189 | 12,3 |
| 190-192 | 50 |
| 193-209 | 218 |
| 210-216 | 23,8 |
| 217-225 | 29,6 |
| 226-231 | 21,4 |
| 232-235 | 8,5 |
| 236-295 | 11 |

### 3) Système hexaméthyldisiloxane / acétonitrile / tétrahydrofurane / eau

### a- Système biphasique de solvants :

Le système utilisé correspond au mélange hexaméthyldisiloxane / acétonitrile / tétrahydrofurane / eau 9:5:2:0,4 (v/v). Les solvants sont agités dans une ampoule à décanter, puis séparés. La phase organique est choisie comme phase mobile, la phase aqueuse comme phase stationnaire.

### b- Séparation par CPC : (expérience 4)

450 mg d'extrait M1(-sucres) obtenu selon la méthode décrite ci-dessus sont dissous dans 10 ml de phase stationnaire aqueuse et 10 ml de phase mobile organique.

La colonne est remplie de phase stationnaire aqueuse (300 tours/min, mode ascendant, 20 ml/min), puis la rotation est fixée à 1 800 tours/min. Le volume d'injection est alors introduit dans la colonne via une boucle d'injection puis poussé par la phase mobile à 5 ml/min. L'élution en mode ascendant est poursuivie pendant 80 minutes, le pourcentage de rétention de la phase stationnaire étant de 55% et la perte de charge de 19 bars. Au bout de 80 minutes, les phases mobiles et stationnaires sont inversées, pour cette fois effectuer une élution en mode descendant ("dual-mode"). Le mode descendant est poursuivi pendant 16 minutes. L'effluent est fractionné grâce à un collecteur automatique de fractions (SuperFrac Pharmacia). La détection s'effectue en UV (210 nm). L'analyse des fractions par CCM à polarité de phases inversée montre que ce système conduit à une séparation intéressante entre les composés d'intérêt, les glycosyglycérides (et les monoacylglycérides) et les autres métabolites secondaires tels que les triglycérides ou les acides gras. Les fractions comprenant les composés d'intérêt et présentant le même profil en CCM sont rassemblées, évaporées sous pression réduite, puis pesées.

Le bilan massique est reporté dans le tableau ci-dessous :

| Tubes rassemblées (35 sec/tube) | Masse en mg |
|---|---|
| 0-19 | 10,5 |
| 20-22 | 7 |
| 23-27 | 8,4 |
| 28-30 | 02,5 |
| 31-33 | 2,1 |
| 34-42 | 11,2 |
| 43-57 | 24,3 |
| 58-70 | 7 |
| 71-93 | 11,8 |
| 94-122 | 14,1 |
| 123-127 | 254,2 |
| 128-130 | 39,1 |
| 131-132 | 11,8 |
| 133-135 | 11 |
| 136-143 | 19,1 |
| 144-166 | 11,6 |

L'extrait M1(-sucres) testé contient entre 70 et 80% de composés d'intérêt, ce qui est en accord avec l'essai précédent.

### III - MISE EN OEUVRE DU PROCÉDÉ AVEC DE LA FARINE DE BLÉ

Matière première : Farine complète production fermière type 110 (21220 Gevrey-Chambertin)

50 g de farine sont mélangés à 50g de sable de Fontainebleau et sont introduits dans une colonne de verre et mis à macérer 48 heures dans 750 ml d'un mélange CHCl₃/MeOH 2:1 v/v. Le solvant est lixivié. L'extrait obtenu est évaporé sous pression réduite pour fournir 1,761 g d'une huile dont le profil en chromatographie sur couche mince est semblable à l'huile obtenue à partir du gluten de blé.

Cette huile peut donc être soumise aux étapes décrites dans le paragraphe précédent.

### IV - PROCÉDÉ D'ENRICHISSEMENT DE L'HUILE DE GLUTEN DE BLÉ PAR DÉPHASAGE

500 g d'huile de gluten de blé sont dissous dans un mélange de solvants composé de 600 ml de chloroforme, 700 ml de méthanol et 500 ml d'eau. La solution est agitée par une palle mécanique à 800 tours/min pendant 5 minutes. Deux phases sont obtenues. La phase supérieure (S0), riche en sucres est évaporée pour fournir 31 g d'un extrait riche en sucres. La phase supérieure est reprise dans un mélange de 400 ml de méthanol et de 200 ml d'eau. Cette nouvelle solution est agité à 800 tours/min pendant 5 minutes pour fournir 550 ml d'une phase supérieure (S1) riche en composés d'intérêts et une phase inférieure I1. L'opération précédente est répétée 2 fois pour fournir 640 ml de S2, 1 260 ml de S3 et I3. I3 est alors dissous dans un mélange composé de 600 ml de méthanol et 200 ml d'eau pour fournir, après agitation à 800 tours/min pendant 1 minute et décantation, 770 ml de phase inférieure S4 et I4. L'opération est répétée à l'identique sur I4 pour fournir 1 320 ml de S5 et 230 ml de I5.

Toutes les solutions sont évaporées sous pression réduite.

**Tableau : Masses des fractions obtenues**

| Phases | Masses (g) |
|---|---|
| S0 | 30,8 |
| S1+S2+S4+S5 | 125,8 |
| S3 | 179,6 |
| I5 | 136,4 |

Les fractions d'intérêts (riches en galactosylglycérides) sont les fractions S1 à S5.

### V - SYNTHÈSE DES GLYCOSYLGLYCÉRIDES

**1- Synthèse des DGDG** (Tanaka et al., Bioorg. Med. Chem., 15 (2005) 159-162)

Ce procédé débute par la préparation du di-galactoside (1→6) bromé en position anomère et peracétylé. La réaction de glycosylation en présence d'HgO et de HgBr₂ dans le dichloro-1,2-éthane est alors effectuée sur la position *sn-3* libre du glycérol benzylé en *sn*-1 et *sn-2.* Une suite réactionnelle classique impliquant des réactions de protection et de déprotection permet d'accéder aux diacylglycérides désirés, les deux chaînes grasses pouvant être différentes.

Autre stratégie : Gent et al, Journal of Chemical Society, Perkin Transactions, 1: Organic and Bioorganic Chemistry, 15, 1975, 1521-1524.

**2 - Synthèse des MGDG** (Nagatsu et al., Bioorgonic & Medicinal Chemistry Letters, Vol. 4. No. 13, pp. 1619-1622, 1994)

Ce procédé met en oeuvre une réaction de glycosylation entre du galactose bromé en position anomère et peracétylé et du glycérol benzylé en position benzylé en *sn-1* et *sn-2,* dans les mêmes condition que ci-dessus. Une suite de réactions de protection et déprotection permet d'individualiser les positions *sn-*1 et *sn-2* du glycérol et de les estérifier par des acides identiques ou non.

**3 - Synthèse des MGMG** (Nagatsu et al., Bioorgonic & Medicinal Chemistry Letters, Vol. 4. No. 13, pp. 1619-1622, 1994)

Le même type de suite réactionnelle que ci-dessus permet également d'accéder à des monogalactosyl-monoacyl-glycérides (position *sn-*2 libre).

### 4 - Synthèse des dérivés du glycérol glycosylés en position sn-2

### Avec un glucose :

Différents composés peuvent être synthétisés à partir du 1,3-di-O-benzyl-2-O-(2,3,4,6-tetra-O-acetyl-β-D-glucopyranosyl)-*sn*-glycerol préparé selon la référence Suhr, R.; Scheel, O.; Thiem, J.; J. Carbohydr. Chem., 1998, 17, 937. Des monoestérifications sélectives en *sn*-3 ou en 6' du glucose peuvent être effectuées par des acylations catalysées par des lipases.

### Avec un galactose :

Le 2-O-β-D-galactoglycérol peut être acylé régio- et stéréo-sélectivement en position 1, 3 ou 6' en utilisant des lipases de *Pseudomonas* ou de *Candidas.* (D. Colombo, F. Ronchetti, A. Scala, L. Toma, Bioactive glycoglycerolipid analogues: an expeditious enzymatic approach to mono-and diesters of 2-O-β-d-galactosylglycerol, Tetrahedron: Asymmetry 9 (1998) 2113-2119).

### 5 - Synthèse des dérivés du glycérol glycosylés avec une configuration α pour l'anomère

Le 2-*O*-α-D-Glucopyranosyl-*sn*-glycérol [R. Suhr, O. Scheel and J. Thiem. J. Carbohydr. Chem. 17 (1998), pp. 937-968] et le 2-*O*-α-D-galactopyranosyl-*sn*-glycérol (4a) [Austin P.W., Hardy F.E., Buchanan J.G., Baddiley J., J Chem. Soc. (1965) 1419-1424.] sont tous les deux préparés par une réaction de glycosylation en appliquant la méthode au trichloroacétimidate [R. Suhr, O. Scheel and J. Thiem., J. Carbohydr. Chem. 17 (1998) pp. 937-968.]. Ils sont directement soumis aux lipases de *Pseudomonas cepacia* (LPS) ou de *Candida antarctica* (LCA) en présence d'hexanoate de 2,2,2-trifluoroéthyl dans la pyridine.

Par ailleurs, tous ces composés répondant à la formule (I) de l'invention peuvent être obtenus par bioconversion ou fermentation à l'aide de microorganismes sélectionnés ou génétiquement modifiés.

### PARTIE EXPÉRIMENTALE - BIOLOGIE

### 1 - Modulation de l'activité de la plasmine, de la MMP-1 et de la MMP-2 in vitro par des glycolipides extraits du gluten de blé

### a - Modulation de l'activité de la plasmine par des glycolipides extraits du gluten de blé

Les analyses par RMN des ces glycolipides ont permis de mettre en évidence la présence d'acides gras insaturés au niveau de leurs chaînes latérales. Comme cela a été montré précédemment, ces acides gras à longue chaîne insaturée sont connus pour interagir avec la plasmine via son domaine kringle V (Huet E, Cauchard JH, Berton A, Robinet A, Decarme M, Hornebeck W, Bellon G. Inhibition of plasmin-mediated prostromelysin-1 activation by interaction of long chain unsaturated fatty acids with kringle 5, Biochem Pharmacol., 2004 Feb 15; 67(4): 643-54. Erratum in: Biochem Pharmacol. 2004). Cette interaction entre l'insaturation de l'acide gras et le domaine kringle V de la plasmine entraîne la perte d'activité de cette dernière. Celle-ci se trouvant à l'origine de la cascade protéolytique de la plasmine, l'inhibition de son activité pourrait alors entraîner une inhibition de l'activation de la proMMP-3 mais également de la proMMP-1, enzymes particulièrement impliquées dans la dégradation de matrice extracellulaire observée au cours des vieillissements cutanés.

L'effet de ces différentes fractions et produits purs a donc été testé *in vitro* sur l'activité amidolytique de la plasmine à l'aide d'un substrat synthétique (S-2251, Chromogénix).

### Mode opératoire détaillé :

Les fractions ou composés purs issus de l'extraction sont solubilisés avant utilisation dans du diméthylsulfoxyde (DMSO) à une concentration de stockage de 20 mg/mL. La conservation est effectuée à -80°C. 12 nM de plasmine humaine sont pré-incubés en absence et en présence de concentrations croissantes (0 à 200 µg/mL) des différentes fractions et produits dans un tampon Tris-HCl 100 mM pH 7,5. La réaction est déclenchée par addition de 0,3 mM de S-2251. L'hydrolyse du S-2251 est suivie par mesure de l'absorbance du para-nitroaniline à 405 nm. La courbe représentant les variations d'absorbance en fonction du temps est tracée et la vitesse initiale de la réaction est déterminée par calcul de la pente de la tangente à l'origine. Le rapport Vi/Vo est calculé, où Vo représente la vitesse initiale en absence d'effecteur et Vi cette vitesse en présence d'un effecteur, et la courbe Vi/Vo en fonction de la concentration en en glycolipides est tracée.

### Résultats :

### Indication de composition des fractions testées (analyse RMN)

| **Fractions testées** | **Composition** |
|---|---|
| ED23-11 | MAMGDG |
| 28-31 (EXP1) | MAG, chaîne grasse en C18:2 |
| 32-39 (EXP1) | MGDG (1 ou 2 C18 :2), acides gras, cires, DGDG |
| 58-67 (EXP1) | DGDG (1 ou 2 C18:2), MGDG |
| 76-80 (EXP1) | DGDG saturé |
| 87-93 (EXP1) | MGDG, DGDG, DGMG |
| 107-112 (EXP1) | MGDG, DGMG |
| 121-124 (EXP1) | DGMG (1 C18:2), MGMG |
| 125-127 (EXP1) | DGMG, MGMG et DGDG minoritaire |
| 43-44 (EXP2) | DGDG, acides gras, MAG |
| 64-66 (EXP2) | MGMG insaturés, MAG |
| 93-100 (EXP2) | DGDG insaturés (1 ou 2 C18 :2) et saturés |
| 125-136 (EXP2) | DGMG, MGDG |
| 155 (EXP2) | DGMG, DGDG minoritaires |
| 40-56 (EXP3) | Acides gras, DGDG, DGMG |

### Résultats sur l'activité amidolytique de la plasmine (tableau 1)

**Tableau 1**

| **Extraits** | **Plasmine (Vi/Vo)** |
|---|---|
| Huile brute (100 µg/mL) | 1,17 ± 0,09 |
| M1 (100 µg/mL) | 1,45 ± 0,21 |
| M1(-Sucres) (50 µg/mL) | 1,79 ± 0,05 |
| M1(-Sucres) (lot 2) (50 µg/mL) | 1,89 ± 0,11 |
| M1(-Sucres) (lot 3) (50 µg/mL) | 1,91 ± 0,09 |
| S1 (50 µg/mL) | 2,98 ± 0,13 |
| S2 (50 µg/mL) | 1,65 ± 0,31 |
| S3 (50 µg/mL) | 1,87 ± 0,14 |
| S4 (50 µg/mL) | 2,01 ± 0,17 |
| S5 (50 µg/mL) | 1,71 ± 0,25 |
| **Fractions obtenues après chromatographie (CPC)** | |
| ED23-11 (25 µg/mL) | 2,11 ± 0,16 |
| 28-31 (EXP 1) (25 µg/mL) | 1,96 ± 0,12 |
| 32-39 (EXP 1) (50 µg/mL) | 2,09 ± 0,06 |
| 58-67 (EXP 1) (100 µg/mL) | 1,74 ± 0,09 |
| 76-80 (EXP 1) (100 µg/mL) | 1,62 ± 0,15 |
| 87-93 (EXP 1) (100 µg/mL) | 1,39 ± 0,14 |
| 107-112 (EXP 1) (100 µg/mL) | 1,44 ± 0,07 |
| 121-124 (EXP 1) (50 µg/mL) | 1,89 ± 0,06 |
| 125-127 (EXP 1) (100 µg/mL) | 1,73 ± 0,24 |
| 43-44 (EXP 2) (100 µg/mL) | 1,76 ± 0,45 |
| 64-66 (EXP 2) (50 µg/mL) | 1,53 ± 0,09 |
| 93-100 (EXP 2) (100 µg/mL) | 2,12 ± 0,21 |
| 125-136 (EXP 2) (100 µg/mL) | 1,95 ± 0,31 |
| 155 (EXP 2) (100 µg/mL) | 2,88 ± 0,09 |
| 40-56 (EXP 3) (100 µg/mL) | 1,45 ± 0,14 |

Les résultats présentés dans le tableau 1 montrent que l'ensemble des fractions et composés purs testés interagit avec la plasmine. En effet, l'ensemble des rapports Vi/Vo calculé apparaît supérieur à 1. Tous les principes actifs testés augmentent l'activité amidolytique de la plasmine, cependant aucun de ces composés ne présente d'activité significativement différente par rapport aux autres. On remarque également que les fractions enrichies en lipides (M1) et désucrées M1(-Sucres) présentent une activité significativement supérieure par rapport à l'huile brute de départ. De plus les fractions M1 et M1(-sucres) provenant de lots de blé différents présentent des Vi/Vo sensiblement identiques.

### b - Modulation de l'activité de MMP-1 par des glycolipides extraits du gluten de blé

La collagénase-1 ou MMP-1 est la principale métalloprotéinase matricielle impliquée dans la dégradation de la matrice collagénique dermique observée lors des processus de vieillissements. Il est donc particulièrement intéressant de s'intéresser à l'effet inhibiteur que présentent les différentes fractions et produits purs de l'invention sur l'activité collagénolytique de la MMP-1. Le criblage des différents composés et fractions a été réalisé *in vitro* à l'aide d'un substrat synthétique fluorescent.

### Mode opératoire détaillé :

La proMMP-1 recombinante (75 ng/mL) est activée par l'APMA (Acide Phényl Mercurique Acétate). L'enzyme active est alors préincubée 10 min en présence des fractions ou composés à tester à différentes concentrations (0 à 200 µg/mL). Le substrat synthétique de la MMP-1 est ensuite ajouté. Le clivage de ce substrat est estimé par mesure de la fluorescence libérée (λ_{exc} = 360 nm ; λₑₘ = 465 nm) à l'aide d'un spectrofluorimètre lecteur de plaques de type Perkin Elmer HTS 7000 Plus. Les intensités de fluorescence obtenues permettent d'estimer la vitesse initiale de l'enzyme en l'absence d'effecteur (Vo) et en présence d'inhibiteur (Vi). Le rapport Vi/Vo est calculé pour chaque concentration de produit et permet de déterminer l'activité de l'enzyme en présence d'effecteur, exprimée en pourcentage de l'activité de l'enzyme seule.

### Résultats :

**Tableau 2 (Résultats sur l'inhibition de l'activité de la MMP-1)**

| **Extraits** | **Inhibition de MMP-1 (%)** | **MMP-1** IC50 (µg/mL) |
|---|---|---|
| Huile brute (100 µg/mL) | 10 ± 0,43 | ∞ |
| M1 (100 µg/mL) | 27,1 ± 1,11 | - |
| M1(-Sucres) (50 µg/mL) | 30,9 ± 0,73 | 197,23 |
| M1 (-Sucres) (lot 2) (50 µg/mL) | 26 ± 1,47 | - |
| M1(-Sucres) (lot 3) (50 µg/mL) | 30 ± 0,83 | - |
| S1 | 30 (50 µg/mL) | 85,32 |
| S2 | 80 (50 µg/mL) | 26,31 |
| S3 | 0 (50 µg/mL) | 103,5 |
| S4 | 50 (50 µg/mL) | 51,06 |
| S5 | 45 (50 µg/mL) | 68,52 |
| **Fractions obtenues après chromatographie (CPC)** | | |
| ED23-11 (25 µg/mL) | 42 ± 0,87 | - |
| 28-31 (EXP 1) (25 µg/mL) | 38 ± 1,56 | - |
| 32-39 (EXP 1) (50 µg/mL) | 8 ± 0,34 | - |
| 58-67 (EXP 1) (100 µg/mL) | 5 ± 1,02 | - |
| 76-80 (EXP 1) (100 µg/mL) | 0 | - |
| 87-93 (EXP 1) (100 µg/mL) | 8 ± 1,21 | - |
| 107-112 (EXP 1) (100 µg/mL) | 12 ± 1,51 | - |
| 121-124 (EXP 1) (50 µg/mL) | 24 ± 3,27 | - |
| 125-127 (EXP 1) (100 µg/mL) | 19 ± 1,84 | - |
| 43-44 (EXP 2) (100 µg/mL) | 9 ± 1,65 | - |
| 64-66 (EXP 2) (50 µg/mL) | 37 ± 2,51 | - |
| 93-100 (EXP 2) (100 µg/mL) . | 8 ± 1,74 | - |
| 125-136 (EXP 2) (100 µg/mL) | 19 ± 0,28 | - |
| 155 (EXP 2) (100 µg/mL) | 12 ± 2,19 | - |
| 40-56 (EXP 3) (100 µg/mL) | 2 ± 0,21 | - |

Les résultats obtenus et présentés dans le tableau 2 montrent un effet inhibiteur de nombreuses fractions notamment des fractions M1(-Sucres) et S1 à S5, sur l'activité de la MMP-1 *in vitro.*

### c - Modulation de l'activité de la MMP-2 par des glycolipides extraits du gluten de blé

L'inhibition de l'activité des gélatinases MMP-2 et MMP-9 par les acides gras insaturés à longue chaîne a pu être montré sur des coupes de peau. Ce phénomène implique à la fois des domaines spécifiques des gélatinases : les "Collagen Binding Domain" (CBD) et la double liaison des acides gras. L'interaction entre les gélatinases, via ces sites particuliers que sont les exosites (CBD), et l'acide gras, via son insaturation, entraîne une perte d'activité des gélatinases *in vitro* comme *in vivo.* La présence de double liaisons au niveau des chaînes latérales de ces glycolipides a conduit à cribler l'ensemble des composés de l'invention sur l'activité de la gélatinase-A ou MMP-2, car cette enzyme, bien qu'elle ne soit pas surexprimée au cours des vieillissements par les cellules dermiques, participe à la dégradation du tissu élastique responsable de la formation de rides. Ce criblage des différentes fractions et composés purs a été réalisé *in vitro* à l'aide d'un substrat synthétique fluorescent.

### Mode opératoire détaillé :

La proMMP-2 recombinante (12,5 ng/mL) est activée par l'APMA 10 mM pendant 16 heures à 4°C puis préincubée 10 min en présence des différentes fractions et composés à tester : le substrat synthétique spécifique de la MMP-2 (Référence : M-1895, Bachem) est ensuite ajouté, à la concentration de 2 µM. Le clivage de ce substrat est estimé par mesure de la fluorescence émise après clivage (λ_{exc} = 326 nm ; λₑₘ = 465 nm), à raison d'une lecture toutes les 2 minutes pendant 30 minutes. Le rapport Vi/Vo est calculé pour chaque concentration de produit.

### Résultats :

**Tableau 3 (Résultats sur l'inhibition de l'activité de la MMP-2)**

| **Extraits** | **MMP-2 (Vi/Vo)** |
|---|---|
| Huile brute (100 µg/mL) | > 1 |
| M1 (100 µg/mL) | > 1 |
| M1(-Sucres) (50 µg/mL) | > 1 |
| M1(-Sucres) (lot 2) (50 µg/mL) | > 1 |
| M1(-Sucres) (lot 3) (50 µg/mL) | > 1 |
| **Fractions obtenues après chromatographie (CPC)** | |
| ED23-11 (25 µg/mL) | > 1 |
| 28-31 (EXP 1) (25 µg/mL) | > 1 |
| 32-39 (EXP 1) (50 µg/mL) | > 1 |
| 58-67 (EXP 1) (100 µg/mL) | > 1 |
| 76-80 (EXP 1) (100 µg/mL) | > 1 |
| 87-93 (EXP 1) (100 µg/mL) | > 1 |
| 107-112 (EXP 1) (100 µg/mL) | > 1 |
| 121-124 (EXP 1) (50 µg/mL) | > 1 |
| 125-127 (EXP 1) (100 µg/mL) | > 1 |
| 43-44 (EXP 2) (100 µg/mL) | > 1 |
| 64-66 (EXP 2) (50 µg/mL) | > 1 |
| 93-100 (EXP 2) (100 µg/mL) | > 1 |
| 125-136 (EXP 2) (100 µg/mL) | > 1 |
| 155 (EXP 2) (100 µg/mL) | > 1 |
| 40-56 (EXP 3) (100 µg/mL) | > 1 |

Les résultats de ce criblage n'ont pas permis de mettre en évidence d'effet inhibiteur de ces différents composés, et ce quelques soient leurs concentrations. En effet, le calcul des rapports des vitesses initiales en présence et en absence d'effecteur (Vi/Vo) donne toujours des résultats supérieurs à 1. L'ensemble des composés testés n'a donc pas d'effet inhibiteur sur l'activité de la MMP-2.

### 2 - Modulation de l'activitation de la proMMP-3 par la plasmine in vitro

Les résultats obtenus précédemment *in vitro* et particulièrement sur la plasmine, ont conduit les Inventeurs à s'intéresser aux effets des différentes fractions et produits de l'invention sur l'activation de la plasmine. En effet, comme décrit antérieurement, les acides gras insaturés peuvent inhiber l'activation de la proMMP-3 par la plasmine (Biochem Pharmacol. 2004 Feb 15;67(4):643-54. Inhibition of plasmin-mediated prostromelysin-1 activation by interaction of long chain unsaturated fatty acids with kringle 5. Huet E, Cauchard JH, Berton A, Robinet A, Decarme M, Hornebeck W, Bellon G).

Les composés de l'invention ont donc été testés pour savoir s'ils sont dotés des mêmes propriétés.

### Mode opératoire :

De la plasmine humaine (65 nM) est incubée en présence de fractions ou composés, à la concentration la plus efficace sur l'activité amidolytique de la plasmine, pendant 15 minutes à 37°C, puis on ajouté 100 nM de proMMP-3 recombinante. La réaction est maintenue pendant 4 heures à 37°C avant d'être stoppée par addition d'aprotinine. Les échantillons sont ensuite analysés par western-blot (immuno-révélation de protéines suite à un électro-transfert) à l'aide d'un anticorps anti-MMP-3.

### Résultats :

La Figure 1 présente les résultats concernant le composé ED 23-11 (MAMGDG), ainsi que la fraction M1(-Sucres). En effet, cette fraction obtenue par une extraction simple et rapide apparaît avoir une activité *in vitro* proche des extraits les plus efficaces, ce qui en terme de développement industriel est intéressant.

Ces résultats confirment les effets observés *in vitro* sur l'activité de la plasmine. En effet, les résultats présentés dans la Figure 1A montrent bien que la fraction M1(-Sucres) ainsi que le composé ED 23-11 interagissent avec la plasmine. La Figure 1A-3 (3^{e} colonne) montre que la fraction M1(-Sucres) à 50 µg/mL inhibe partiellement l'activation de la proMMP-3 par la plasmine. Cette inhibition apparaît cependant moins importante que dans le cas d'une inhibition par le composé ED 23-11 à 25 µg/mL.

### 3 - Influence d'extraits du gluten de blé sur l'activation de la proMMP-3 et de la proMMP-1 par des fibroblastes dermiques en culture ex vivo

Les résultats *in vitro* précédents ont été confirmés en culture cellulaire après vérification de la non-toxicité de ces principes aux concentrations utilisées.

### Mode opératoire :

Les fibroblastes dermiques cultivés en plaques 24 puits jusque confluence, sont après une mise au repos de 18 heures, incubés en présence ou en absence de 2,5 µg/mL de plasmine ainsi qu'en présence ou non du composé ED 23-11 et de la fraction M1(-Sucres) aux mêmes concentrations que précédemment.

### Résultats :

Les résultats obtenus montrent une reproductibilité de l'activation de la proMMP-3 par la plasmine (figure 1B-2) mais également une activation importante de la proMMP-1 en MMP-1 (figure 1C-2). Cette activation de la collagénase interstitielle est la résultante finale de l'activation de la cascade de la plasmine.

En ce qui concerne les extraits glycolipidiques testés, on note comme dans le cas de l'analyse *in vitro* (figure 1A) une inhibition significative de l'activation de la proMMP-3 par la fraction M1(-sucres) ainsi que par le composé ED 23-11. L'inhibition par ce dernier composé apparaissait supérieure à l'inhibition engendrée par la fraction *in vitro* mais elle apparaît sensiblement équivalente en culture cellulaire (figures 1B-3 et 1B-4).

Au niveau de l'activation de la proMMP-1 (figure 1C), on observe une activation partielle de cette collagénase en réponse à la plasmine (figure 1C-2). Et comme dans le cas de la stromélysine, on observe une inhibition significative de cette activation lorsque les fibroblastes dermiques sont cultivés en présence de la fraction M1(-Sucres)(figure 1C-3), ou du composé ED 23-11 (figure 1C-4).

### 4 - Effets des fractions glycolipidiques sur la dégradation d'une matrice de collagène dans un modèle de photo-vieillissement

Après avoir mis en évidence l'efficacité de la fraction M1(-sucres) dans la régulation de la cascade de la plasmine ainsi que sur l'activation de la pro collagénase-1, les Inventeurs se sont intéressés à l'efficacité de ces glycolipides dans un modèle de photo-vieillissement cutané. En effet dans ce modèle, il a été mis en évidence précédemment, une importante formation de plasmine ainsi que l'activation des stomélysine-1 et collagénase-1 avec pour conséquence la dégradation de la matrice collagénique, support de cette co-culture.

L'efficacité du composé ED 23-11 à la concentration de 25 µg/mL, ainsi que celle de la fraction M1-Sucres à 50 µg/mL ont donc été testées sur la dégradation de cette matrice de collagène.

### Mode opératoire :

Pour cela 2 mL d'une suspension de kératinocytes et de fibroblastes dermiques sont ensemencés en plaque 6 puits sur un tapis de collagène de type I à 300 µg/mL marqué à la fluorescéine. Après 3 heures d'adhésion en milieu contenant 2% de sérum, les cellules sont rincées abondamment puis photo-irradiées par 10 J.cm⁻² d'UV-A. Le milieu est ensuite supplémenté par 15 µg/mL de plasminogène ainsi que par les glycolipides de l'invention, c'est-à-dire par M(-Sucres) à 50 µg/mL (Figure 2A-3 et 2B-3) et par ED 23-11 à 25 µg/mL (Figure 2A-4 et 2B-4) . Les cellules sont maintenues en culture pendant 48 heures, puis la dégradation du tapis de collagène est mesurée. Les milieux de culture sont récupérés, puis 100 µL de chaque échantillon sont analysés par spectrofluorimétrie avec des longueurs d'onde d'excitation de 544 nm et d'émission de 590 nm.

### Résultats :

Les résultats présentés dans la figure 2 mettent en évidence un effet inhibiteur de la fraction M1(-Sucres) (Figure 2A-3 et 2B-3) et du composé ED 23-11 (Figure 2A-4 et 2B-4) sur la dégradation du tapis de collagène liée au photo-vieillissement cutané. Cette inhibition est mise en évidence par une diminution de la fluorescence "relarguée" dans le milieu de culture après 48 heures de culture post-irradiation. La mesure de la fluorescence libérée permet de rendre ce modèle de vieillissement quantifiable.

L'inhibition de ce "vieillissement cutané" par la fraction M1(-Sucres) est de 55% et celle du composé ED 23-11 de 70% par rapport au témoin sans principe actif (voir Figure 2B).

### 5 - Effets de la fraction M1(-Sucres) et du composé ED23-11 sur la synthèse des collagènes totaux

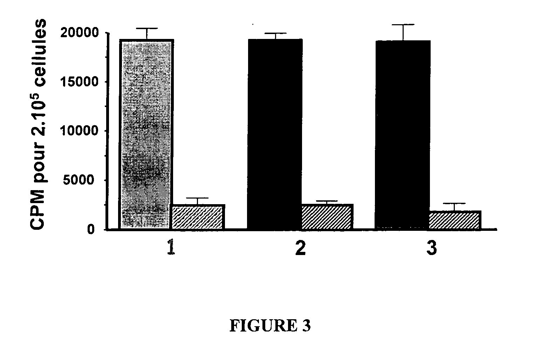

2.10⁵ fibroblastes dermiques par puits sont ensemencés en plaque 6 puits jusque confluence, puis incubés pendant 24 heures en condition témoin (Figure 3-1) ou en présence de la fraction M1(-Sucres) (Figure 3-2) à 50 g/mL ou du composé ED 23-11 (Figure 3-3) à 25 µg/mL. L'incorporation de proline-H³ dans les protéines permet de mesurer la synthèse des protéines collagéniques au niveau cellulaire comme dans le milieu de culture. L'incorporation de proline-³H est mesurée à la fois dans le surnageant et au niveau cellulaire.

Aucune différence significative n'est observée sur la synthèse des collagènes totaux et ce, quelque soit la nature de l'effecteur testé. Les composés de l'invention ne présentent donc pas d'activité sur la synthèse des collagènes et ne permettent donc pas de participer à la resynthèse de la matrice collagénique du derme dégradée au cours des vieillissements cutanés.

### 6 - Effets de la fraction M1(-sucres) du composé ED 23-11 et de DGDG commercial sur la sécrétion de TIMP-1

Le composé commercial (Ref: 1058, Matraya) présente la formule suivante: C₅₁H₉₆O₁₅

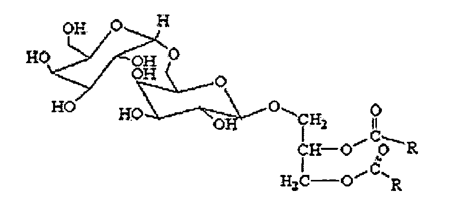

Les chaînes latérales grasses sont de type : stéaroyle

Les effets de ces composés et fractions ont été étudiés sur la sécrétion de TIMP-1 par des fibroblastes dermiques en culture. L'analyse de la quantité de TIMP-1 produite est effectuée par une technique de zymographie inverse *(*Biotechniques. 2005 Jan;38(1):73-83. Zymographic techniques for the analysis of matrix metalloproteinases and their inhibitors. Snoek-van Beurden PA, Von den Hoff JW). Les résultats présentés sont exprimés en pourcentage par rapport au témoin sans effecteur.

| Concentration (ng/mL) | 0 | 5 | 25 | 50 | 100 | 200 |
|---|---|---|---|---|---|---|
| DGDG | 100 | 107 | 121 | 135 | 186 | 199 |
| ED 23-11 | 100 | 102 | 104 | 98 | 101 | 106 |
| M1(-Sucres) | 100 | 95 | 93 | 97 | 106 | 104 |

D'après ce tableau, on constate que le DGDG entraîne une surexpression significative du TIMP-1 de façon dose dépendante mais pas le composé ED23-11 ni la fraction M1 (-sucres).

Ainsi, les composés de l'invention présentent une activité d'inhibition direct des métalloprotéinases, indépendante de TIMP-1.

### 7 - Effets du DGDG commercial sur l'activité de la MMP-1

L'effet de ce DGDG commercial sur l'activité de la MMP-1 a été déterminé par une technique de FRET suivant le protocole décrit ci-dessus.

Les résultats sont exprimés en pourcentage par rapport au témoin sans effecteur.

| Concentration (ng/mL) | 0 | 5 | 10 | 25 | 50 | 100 | 200 |
|---|---|---|---|---|---|---|---|
| DGDG | 100 | 98 | 101 | 111 | 105 | 99 | 102 |

On constate donc que le DGDG commercial n'inhibe pas l'activité de la MMP-1 sur substrat synthétique.

### 8 - Effet du DGDG commercial sur l'activité de la plasmine

Cet effet a été déterminé par activation de la proMMP-3 en MMP-3 par la plasmine suivant le protocole détaillé ci-dessus.

Le DGDG a été testé aux concentrations suivantes : 10, 50 et 100 µg/mL.

On a alors constaté que le DGDG n'inhibe pas l'activité de la plasmine *in vitro.*

### EXEMPLES DE FORMULATIONS

### 1 - Crème anti-âge

### a - Formule

| Phase | Ingrédient | % |
|---|---|---|
| | Emuliance® (Soliance SA, Pomacle, France) | 4 |
| | Miglyol 812N (Sasol, USA) | 3 |
| A | lsostéarate d'isostéaryle | 3 |
| | Diméthicone 200 (350cps) | 2 |
| | Huile de noyau d'abricot raffinée | 2 |
| | Acide stéarique | 2 |
| | | |
| | Phyliance® (Soliance SA, Pomacle, France) | 0,02 |
| | Eau osmosée | OSP 100 |
| B | NaOH 20% pH<ou=7 | 0,35 |
| | Glycérine | 5 |
| | Hydr'A3 (Soliance SA, Pomacle, France) | 5 |
| | Phénonip (Clariant, Suisse) | 0,5 |
| | | |
| C | Fraction M1-sucres | 0,02 |
| | | |
| D | Parfum | qs |
| | Colorant | qs |
| | | 100 |

### b - Procédé de préparation

a. Peser séparément les phases A et B.
b. Chauffer les phases A et B à 75°C.
c. Additionner lentement B sur A sous émulseur à 3000 tpm (Polytron PT 300) pendant 5 min.
d. Laisser refroidir sous agitation mécanique (pale).
e. A 35°C, ajouter les phases C et D.

### 2 - Gel visage anti-âge

### a - Formule

| Phase | Ingrédient | % |
|---|---|---|
| | Phénonip (Clariant, Suisse) | 0,4 |
| A | Fraction M1(-sucres) | 0,02 |
| | Parfum | qs |
| | Tween 20 | 1 |
| B | Natrosol 250 HHR (Hercules, USA) | 1 |
| | Eau osmosée | QSP 100 |
| | | |
| C | Colorant | qs |
| | | |
| D | Triéthanolamine qs pH=5,5 | 0,025 |

### b - Procédé de préparation

a. Peser et mélanger les ingrédients de la phase A jusqu'à l'obtention d'un mélange homogène.
b. Peser et mélanger à 40°C les ingrédients de la phase B jusqu'à solubilisation du Natrosol 250 HHR.
c. Additionner lentement A sur B.
d. Ajouter les phases C et D.

### 3 - Microencapsulation de la fraction M1(-sucres) (sphérulites)

### a - Formule

| Phase | Ingrédient | % |
|---|---|---|
| A | Tween 60 | 11,375 |
| | Stéarate de sorbitan | 11,375 |
| | Fraction M1(-sucres) | 4 |
| | Phénonip (1) (Clariant, Suisse) | 0,25 |
| B | Eau déminéralisée (1) | 23 |
| C | Eau déminéralisée (2) | 49,5 |
| | Phénonip (2) (Clariant, Suisse) | 0,5 |

### b - Procédé de préparation

a. Peser et mélanger (Planétaire : 50 tpm) l'ensemble de la phase A à température ambiante.
b. Mettre sous vide (-0,5 bar) et chauffer à T_{cuve} = 65°C. Quand T_{cuve} = 55°C mettre en route la turbine à 900 tpm.
c. Ajouter la phase B par le vide à Tcuve= 65°C. Augmenter la turbine à 1000 tpm pendant 15 minutes.
d. refroidir à 35°C (Turbine : 1000 tpm ; Planétaire : 50 tpm)
e. Disperser les sphérulites avec les ingrédients de la phase C pendant 1 heure jusqu'à l'obtention d'un mélange homogène. (Turbine : 1000 tpm ; Planétaire : 50 tpm)

### 4 - Crème contenant les sphérulites M1(-sucres)

### a - Formule

| Phase | Ingrédient | % |
|---|---|---|
| | Emuliance® (Soliance SA, Pomacle, France) | 4 |
| | Miglyol 812N (Sasol, USA) | 3 |
| A | lsostéarate d'isostéaryle | 3 |
| | Diméthicone 200 (350cps) | 2 |
| | Huile de noyau d'abricot raffinée | 2 |
| | Acide stéarique | 2 |
| | | |
| | Phyliance® (Soliance SA, Pomacle, France) | 0,02 |
| | Eau osmosée | QSP 100 |
| B | NaOH 20% pH<ou=7 | 0,35 |
| | Glycérine | 5 |
| | Hydr'A3 (Soliance SA, Pomacle, France) | 5 |
| | Phénonip (Clariant, Suisse) | 0,5 |
| | | |
| C | Sphérulites M1-sucres | 0,5 |
| | | |
| D | Parfum | qs |
| | Colorant | qs |
| | | 100 |

### b - Procédé de préparation

f. Peser séparément les phases A et B.
g. Chauffer les phases A et B à 75°C.
h. Additionner lentement B sur A sous émulseur à 3000 tpm (Polytron PT 300) pendant 5 min.
i. Laisser refroidir sous agitation mécanique (pale).
j. A 35°C, ajouter les phases C et D.

## Revendications

1. Utilisation d'au moins un composé répondant à la formule (I) suivante : 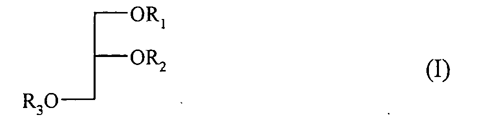 dans laquelle :
― R₁ représente un atome d'hydrogène ou un groupe acyle, saturé ou non, comprenant de 2 à 20 atomes de carbone,
― R₂ représente un atome d'hydrogène, un groupe acyle, saturé ou non, comprenant de 2 à 20 atomes de carbone, ou un groupe de formule suivante (A): 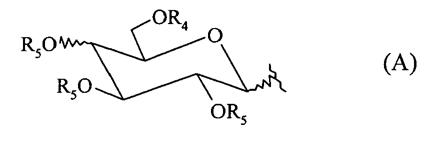 dans laquelle :
* R₄ représente un atome d'hydrogène, un groupe acyle, saturé ou non, comprenant de 2 à 20 atomes de carbone, ou un groupe de formule (B) suivante : 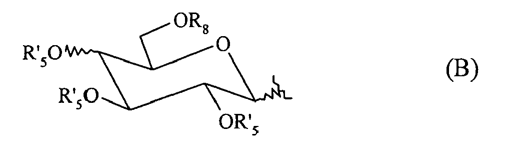
. R'₅ représentant un atome d'hydrogène ou un groupe acyle, saturé ou non, comprenant de 2 à 20 atomes de carbone, et
. R₈ représentant un atome d'hydrogène ou un groupe acyle, saturé ou non, comprenant de 2 à 20 atomes de carbone,
* R₅ représente un atome d'hydrogène ou un groupe acyle, saturé ou non, comprenant de 2 à 20 atomes de carbone,
― R₃ représente un atome d'hydrogène ou un groupe de formule suivante (C) : 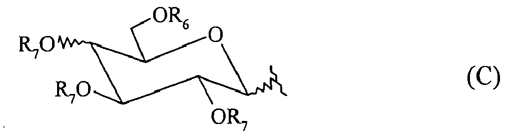 dans laquelle :
* R₆ représente un atome d'hydrogène, un groupe acyle, saturé ou non, comprenant de 2 à 20 atomes de carbone, ou un groupe de formule (B) telle que définie ci-dessus,
* R₇ représente un atome d'hydrogène ou un groupe acyle, saturé ou non, comprenant de 2 à 20 atomes de carbone,
pour la préparation d'une composition cosmétique, d'une composition pharmaceutique destinée au traitement des cancers n'impliquant pas le virus EBV, notamment destinée au traitement des mélanomes, ou destinée au traitement des processus inflammatoires de la sphère buccale, ou d'une composition nutraceutique,
. sous réserve que lorsque ledit composé est un composé de la famille des di-galactosyl-di-glycérides répondant à la formule (1) suivante : 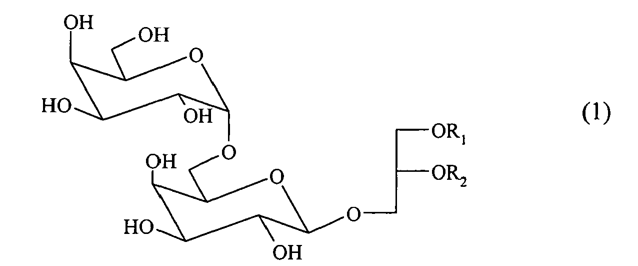 ladite composition cosmétique, pharmaceutique ou nutraceutique contient au moins un deuxième composé de formule (I) différent de celui de formule (1),
. et sous réserve que, dans la formule (I), lorsque R₁ et R₂ représentent un atome d'hydrogène, R₃ représente un groupe de formule (C),
. et sous réserve que, dans la formule (I), lorsque R₁ et R₂ représentent un groupe acyle, R₃ représente un groupe de formule (C),
. et sous réserve que les composés de formule (I) sont présents dans ladite composition cosmétique, pharmaceutique ou nutraceutique à des doses telles qu'ils ne présentent pas d'activité sur la fibronectine.

2. Composition cosmétique, notamment destinée à une application topique, contenant au moins un composé de formule (I) suivante : 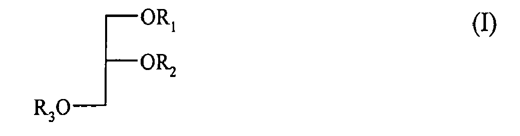 dans laquelle :
― R₁ représente un atome d'hydrogène ou un groupe acyle, saturé ou non, comprenant de 2 à 20 atomes de carbone,
― R₂ représente un atome d'hydrogène, un groupe acyle, saturé ou non, comprenant de 2 à 20 atomes de carbone, ou un groupe de formule suivante (A): 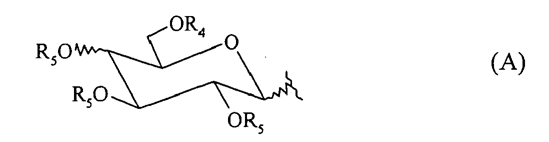 dans laquelle :
* R₄ représente un atome d'hydrogène, un groupe acyle, saturé ou non, comprenant de 2 à 20 atomes de carbone, ou un groupe de formule (B) suivante : 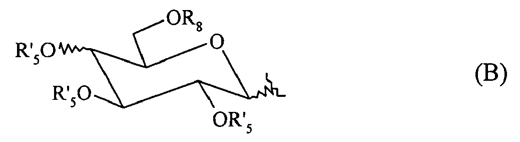
. R'₅ représentant un atome d'hydrogène ou un groupe acyle, saturé ou non, comprenant de 2 à 20 atomes de carbone, et
. R₈ représentant un atome d'hydrogène ou un groupe acyle, saturé ou non, comprenant de 2 à 20 atomes de carbone,
* R₅ représente un atome d'hydrogène ou un groupe acyle, saturé ou non, comprenant de 2 à 20 atomes de carbone,
― R₃ représente un atome d'hydrogène ou un groupe de formule suivante (C) : 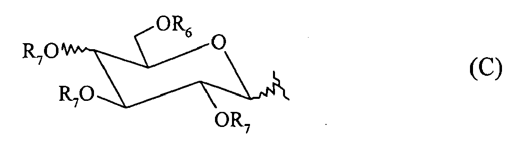 dans laquelle :
* R₆ représente un atome d'hydrogène, un groupe acyle, saturé ou non, comprenant de 2 à 20 atomes de carbone, ou un groupe de formule (B) telle que définie ci-dessus,
* R₇ représente un atome d'hydrogène ou un groupe acyle, saturé ou non, comprenant de 2 à 20 atomes de carbone,
. sous réserve que lorsque ledit composé est un composé de la famille des di-galactosyl-di-glycérides répondant à la formule (1) suivante : 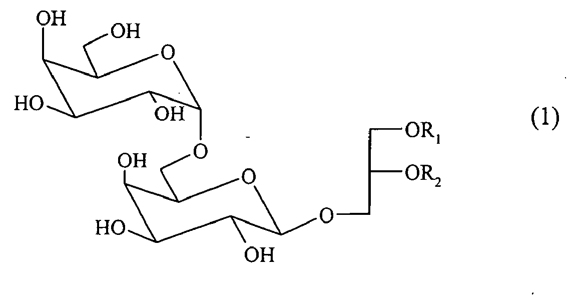 ladite composition cosmétique contient au moins un deuxième composé de formule (I) différent de celui de formule (1),
. et sous réserve que, dans la formule (I), lorsque R₁ et R₂ représentent un atome d'hydrogène, R₃ représente un groupe de formule (C),
. et sous réserve que, dans la formule (I), lorsque R₁ et R₂ représentent un groupe acyle, R₃ représente un groupe de formule (C),
. et sous réserve que les composés de formule (I) sont présents dans ladite composition cosmétique à des doses telles qu'ils ne présentent pas d'activité sur la fibronectine,
en association avec un véhicule approprié.

3. Composition cosmétique selon la revendication 2, **caractérisée en ce que** l'un au moins des groupes R₁ et R₂ représente un groupe acyle tel que défini dans la revendication 2.

4. Composition cosmétique selon la revendication 2, **caractérisée en ce que** R₁ et R₂ représentent un groupe acyle tel que défini dans la revendication 2.

5. Composition cosmétique selon la revendication 2, **caractérisée en ce qu'**elle comprend au moins un composé de formule (I) dans laquelle :
― R₁ représente un groupe acyle, saturé ou non, comprenant de 2 à 20 atomes de carbone, et représente de préférence un groupe linoléyle, palmitoléyle, palmityle ou stéaryle, ou R₁ représente un atome d'hydrogène,
― R₂ représente un atome d'hydrogène lorsque R₁ représente un groupe acyle tel que défini ci-dessus, ou R₂ représente un groupe acyle, saturé ou non, comprenant de 2 à 20 atomes de carbone, et de préférence un groupe linoléyle, palmitoléyle, palmityle ou stéaryle, lorsque R₁ représente un atome d'hydrogène, et
― R₃ représente un atome d'hydrogène.

6. Composition cosmétique selon la revendication 2, **caractérisée en ce qu'**elle comprend au moins un composé de formule (I) dans laquelle :
― R₁ représente un atome d'hydrogène ou un groupe acyle, saturé ou non, comprenant de 2 à 20 atomes de carbone, de préférence un groupe linoléyle, palmitoléyle, palmityle ou stéaryle,
― R₂ représente un atome d'hydrogène lorsque R₁ représente un groupe acyle tel que défini ci-dessus, ou R₂ représente un groupe acyle, saturé ou non, comprenant de 2 à 20 atomes de carbone, de préférence un groupe linoléyle, palmitoléyle, palmityle ou stéaryle, lorsque R₁ représente un atome d'hydrogène,
― R₃ représente un groupe de formule (C) telle que définie dans la revendication 2, dans laquelle R₆ représente un atome d'hydrogène.

7. Composition cosmétique selon la revendication 2, **caractérisée en ce qu'**elle comprend au moins un composé de formule (I) dans laquelle :
― R₁ représente un groupe acyle, saturé ou non, comprenant de 2 à 20 atomes de carbone, et de préférence un groupe linoléyle,
― R₂ représente un groupe acyle, saturé ou non, comprenant de 2 à 20 atomes de carbone, et de préférence un groupe linoléyle, et
― R₃ représente un groupe de formule (C) telle que définie dans la revendication 2, dans laquelle R₆ représente un atome d'hydrogène.

8. Composition cosmétique selon la revendication 2, **caractérisée en ce qu'**elle comprend au moins un composé de formule (I) dans laquelle :
― R₁ et R₂ représentent un groupe acyle, saturé ou non, comprenant de 2 à 20 atomes de carbone, et de préférence un groupe linoléyle, et
― R₃ représente un groupe de formule (C) telle que définie dans la revendication 2, dans laquelle R₆ représente un groupe acyle, saturé ou non, comprenant de 2 à 20 atomes de carbone.

9. Composition cosmétique selon la revendication 2, **caractérisée en ce qu'**elle comprend au moins un composé de formule (I) dans laquelle :
- R₁ représente un atome d'hydrogène, ou R₁ représente un groupe acyle, saturé ou non, comprenant de 2 à 20 atomes de carbone, et de préférence un groupe linoléyle,
- R₂ représente un atome d'hydrogène lorsque R₁ représente un groupe acyle tel que défini ci-dessus, ou R₂ représente un groupe acyle, saturé ou non, comprenant de 2 à 20 atomes de carbone, et de préférence un groupe linoléyle, lorsque R₁ représente un atome d'hydrogène, et
- R₃ représente un groupe de formule (C) telle que définie dans la revendication 3, dans laquelle R₆ représente un groupe de formule (B) telle que définie dans la revendication 3.

10. Composition cosmétique selon la revendication 2, **caractérisée en ce qu'**elle comprend au moins un composé de formule (I) dans laquelle :
― R₁ représente un groupe acyle, saturé ou non, comprenant de 2 à 20 atomes de carbone, et de préférence un groupe linoléyle,
― R₂ représente un groupe acyle, saturé ou non, comprenant de 2 à 20 atomes de carbone, et de préférence un groupe linoléyle, et
― R₃ représente un groupe de formule (C) telle que définie dans la revendication 3, dans laquelle R₆ représente un groupe de formule (B) telle que définie dans la revendication 3.

11. Composition cosmétique selon la revendication 2, **caractérisée en ce qu'**elle comprend au moins un composé de formule (I) dans laquelle R₁ et/ou R₂ représente(nt) un groupe acyle comprenant au moins une insaturation.

12. Composition cosmétique selon l'une quelconque des revendications 2 à 11, **caractérisée en ce que** les composés de formule (I) présentent une activité d'inhibition des métalloprotéinases matricielles, notamment d'inhibition des collagénases.

13. Méthode de traitement cosmétique, comprenant l'administration d'une quantité appropriée d'une composition cosmétique selon l'une quelconque des revendications 2 à 12.

14. Procédé de préparation d'une composition cosmétique telle que définie dans la revendication 2 et contenant des fractions lipidiques enrichies en composés de formule (I) ou des sous-fractions contenant au moins un composé de formule (I), ledit procédé comprenant les étapes suivantes :
― une étape d'enrichissement d'une huile concentrée en composés de formule (I), à raison d'environ 50% à environ 95%,
. d'une part, par élimination de tout ou partie des composés apolaires, tels que les triglycérides, les stérols, les cires, les acides gras et autres composés insaponifiables, notamment par partage liquide/liquide de ladite huile concentrée, à l'aide d'un système biphasique de solvants ou par simple déphasage dans un mélange hydroalcoolique, éventuellement en présence d'un solvant organique tel que le chloroforme ou le méthylterbutyléther, afin d'obtenir une phase liquide enrichie en composé apolaires susmentionnés et une fraction lipidique liquide enrichie en composés de formule (I), notamment exempte de triglycérides,
. et, éventuellement, d'autre part, par élimination des composés polaires tels que les sucres résiduels, notamment par partage liquide/liquide de ladite fraction lipidique enrichie en composés de formule (I) telle qu'obtenue précédemment, à l'aide d'un système biphasique de solvants ou par simple déphasage dans un mélange hydroalcoolique, éventuellement en présence d'un solvant organique tel que le chloroforme ou le méthylterbutyléther, ce qui permet d'obtenir une fraction lipidique enrichie en composés de formule (I), exempte de sucres,
l'ordre des étapes d'élimination des composés apolaires et des composés polaires pouvant être inversé,
― et, éventuellement, une étape de fractionnement de ladite fraction lipidique enrichie en composés de formule (I) ou de ladite fraction lipidique enrichie en composés de formule (I), exempte de sucres, notamment par chromatographie sur gel de silice, par chromatographie liquide sur support solide ou par chromatographie de partage centrifuge, éventuellement par fluides supercritiques, afin d'isoler des sous-fractions contenant au moins un composé de formule (I).

15. Procédé de préparation selon la revendication 14, **caractérisé en ce que** l'élimination de tout ou partie des composés apolaires comprend les étapes suivantes :
― une étape d'extraction liquide/liquide de l'huile concentrée telle que définie dans la revendication 14, avec un système biphasique à base de méthanol, d'heptane et d'eau, ce qui permet l'obtention d'une fraction dans l'heptane, nommée H1a, et d'une fraction méthanolique, nommée M1a, enrichie en composés de formule (I),
― une étape d'extraction liquide/liquide de ladite fraction M1a avec de l'heptane, ce qui permet l'obtention d'une fraction dans l'heptane, nommée H1b, et d'une fraction méthanolique, nommée M1b, qui correspond à une fraction lipidique enrichie en composés de formule (I), et
― éventuellement, une étape d'évaporation et une étape de dissolution dans de l'heptane desdites fractions H1a et H1b, et une étape d'extraction liquide/liquide desdites fractions dans l'heptane avec du méthanol additionné d'eau, ce qui permet l'obtention d'une fraction dans l'heptane, nommée H2, et d'une fraction méthanolique, nommée M2, enrichie en composés de formule (I).

16. Procédé selon la revendication 15, **caractérisé en ce que** la fraction M1b telle que définie dans la revendication 15 est soumise à une étape d'élimination des composés polaires tels que les sucres résiduels, notamment par partage liquide/liquide dans le système n-butanol/eau, de ladite fraction M1b, ce qui permet d'obtenir une fraction lipidique enrichie en composés de formule (I), exempte de sucres.

17. Procédé selon la revendication 15, comprenant une étape de fractionnement de la fraction H2 telle que définie dans la revendication 15, par chromatographie sur gel de silice, afin d'isoler une sous-fraction contenant au moins un composé de formule (I), dans laquelle R₁ et R₂ représentent un groupe acyle et R₃ représente un groupe de formule (C) telle que définie dans la revendication 2.

18. Procédé selon la revendication 16, comprenant une étape de fractionnement de la fraction lipidique enrichie en composés de formule (I), exempte de sucres, par chromatographie de partage centrifuge, en utilisant les systèmes biphasiques suivants : heptane/acétate d'éthyle/acétone/méthanol/eau et heptane/acétonitrile/n-butanol.
